(19) Europäisches Patentamt

European Patent Office

Office européen des brevets

(11) **EP 1 149 906 A1**

(12) **EUROPEAN PATENT APPLICATION**

(43) Date of publication:
**31.10.2001 Bulletin 2001/44**

(21) Application number: **00108075.3**

(22) Date of filing: **25.04.2000**

(51) Int Cl.$^7$: **C12N 15/12**, C07K 14/715, C07K 16/28, A01K 67/027, A61K 48/00, A61K 38/17, A61K 38/10, A61K 31/70, A61K 39/395, G01N 33/68, C12Q 1/68

(84) Designated Contracting States:
**AT BE CH CY DE DK ES FI FR GB GR IE IT LI LU MC NL PT SE**
Designated Extension States:
**AL LT LV MK RO SI**

(71) Applicant: **Pliva, Farmaceutska, Industrija, Dionicko Drustvo**
**10000 Zagreb (HR)**

(72) Inventors:
• **Naranda, Tatjana, Dr.**
**Mountain View, CA 94043 (US)**
• **Olssen, Lennart, Dr.**
**Mountain View, CA 94043 (US)**

(74) Representative: **Schrell, Andreas, Dr. et al**
**Gleiss & Grosse,**
**Patentanwaltskanzlei,**
**Maybachstrasse 6 A**
**70469 Stuttgart (DE)**

(54) **Thrombopoietin receptor modulating peptide**

(57) The present invention relates to novel diagnostic and pharmaceutical compositions enabling the treatment of thrombocytopenia.

**EP 1 149 906 A1**

Printed by Jouve, 75001 PARIS (FR)

**Description**

The present invention relates to an oligopeptide with the biological activity of a thrombopoietin (TPO) receptor modulating compound, nucleotide sequences encoding the oligopeptide, vectors comprising the nucleotide sequences, host cells comprising the vectors, antibodies reactive with the oligopeptides, pharmaceutical and diagnostic compositions comprising the oligopeptides, nucleotide sequences, antibodies and/or host cells as well as to methods for genetically modifying a cell, methods for modulating the activity of a TPO receptor (TPO-R) and methods to screen for further TPO receptor modulating compounds.

Thrombocytopenia is a widespread, serious and life-threatening disease, occurring both as a primary hematological and as an induced disorder. Patients suffering from severe thrombocytopenia are at a serious risk of spontaneous haemorrhage. Induced thrombocytopenia may be caused by e.g. bone marrow transfusions, cancer chemotherapy, irradiation or allergic reactions. About 50 percent of all new cancer patients diagnosed undergo some form of chemotherapy and receive platelet transfusions. Thrombocytopenia affects at least 25 percent of all patients undergoing chemotherapy. Because repeated cycles of dose-intensive chemotherapy deplete blood platelets which prevent bleeding and aid in the repair of damaged blood vessels, cancer treatments very often must be interrupted to allow platelet counts to recover. The failure of the platelet count to recover causes delay and/or limits use of subsequent cycles of chemotherapy, diminishing the chances of successful treatment.

Treatment of thrombocytopenia is primarily carried out by transfusion of fresh platelet preparations, which are very expensive and not as readily available as a drug, that by injection or oral administration, could increase the number of platelets to the normal level. Each year in the US alone approximately 8 million units of platelets are transfused into patients to reduce the risk of severe bleeding. At least 30 percent of the transfusions result in complications, usually febrile reactions, but occasionally bacteremia, graft-versus-host disease, or acute pulmonary injury. In 15 to 25 percent of patients who require repeated platelet transfusions, incremental platelet responses are inadequate as a result of HLA alloimmunization. In addition, platelet transfusions are expensive. In particular, chemotherapy-induced thrombocytopenia is currently managed by platelet transfusion and/or reducing or delaying chemotherapy until the platelet count increases. Transfusions, however, may place patients at a risk for blood-borne infections such as hepatitis B and hepatitis C, HIV infections and human T-lymphotropic virus, or for immune reactions such as fever. Reducing chemotherapy doses and delaying or stopping treatment can theoretically allow cancerous cells to grow or spread. Thus, there is an urgent need for designing effective drugs for the treatment of thrombocytopenia, which, however, requires detailed understanding of its molecular and biochemical causes.

Megakaryocytes are bone marrow-derived cells, which are responsible for producing circulating blood platelets. Although comprising only a small portion of the bone marrow cells in most species, they have over 10 times the volume of typical marrow cells. Megakaryocytes undergo endomitosis, whereby they replicate their nuclei but fail to divide their cells, and thereby give rise to polyploid cells. In response to a decreased platelet count, the endomitotic rate increases, higher ploidy megakaryocytes are formed, and the number of megakaryocytes may increase up to threefold. In contrast, in response to an elevated platelet count, the endomitotic rate decreases, lower ploidy megakaryocytes are formed, and the number of megakaryocytes may significantly decrease.

The exact physiological feedback mechanism by which the mass of circulating platelets regulate the endomitotic rate and number of bone marrow megakaryocytes is not known. The circulating thrombopoietic factor involved in mediating this feedback loop is now thought to be TPO, a glycoprotein occurring in at least two forms having molecular masses of 25 and 31 KDa, with a common N-terminus and regulating red blood cell production. TPO was shown to be a major regulator of megakaryocytopoiesis, both in vivo and in vitro. TPO initiates its biological effects by binding to c-mpl (in the following also termed TPO-R) which is a member of the hematopoietin receptor superfamily. More specifically, TPO has been shown to be the main humoral regulator in situations involving thrombocytopenia. TPO has been shown in several studies to increase platelet counts, increase platelet size, and increase isotope incorporation into platelets of recipient animals. Specifically, TPO is thought to affect megakaryocytopoiesis in several ways: (1) it produces increases in megakaryocyte size and number; (2) it produces an increase in DNA content, in the form of polyploidy, in megakaryocytes; (3) it increases megakaryocyte endomitosis; (4) it produces increased maturation of megakaryocytes; and (5) it produces an increase in the percentage of precursor cells, in the form of small acetylcholinesterase-positive cells and in the bone marrow.

Because platelets (thrombocytes) are necessary for blood clotting, and when their numbers are very low a patient is at serious risk of death from catastrophic haemorrhage, TPO has potential useful applications in both the diagnosis and the treatment of various hematological disorders, for example, diseases primarily due to platelet defects. In addition, recent studies have provided a basis for the projection of efficacy of TPO therapy in the treatment of thrombocytopenia, and particularly thrombocytopenia resulting from chemotherapy, radiation therapy, or bone marrow transplantation as a treatment for cancer or lymphoma, (McDonald (1992) Am. J. Ped. Hematology/Oncology 14:8-21).

The gene encoding TPO has been cloned and characterised (Kuter et al. (1994) Proc. Natl. Acad. Sci. USA 91:11104-11108; Barley et al. (1994) Cell 77:1117-1124; Kaushansky et al. (1994) Nature 369:568-571; Wendling et al. (1994) Nature 369:571-574; and Savage et al. (1994) Nature 369:533-538).

Recombinant thrombopoietin (in the following also termed rTPO) is the only specifically designed compound obtained to date which is possibly effective for the treatment of thrombocytopenia. It acts as a platelet-inductor drug during thrombocytopenia. It has been shown that the exogenous single dose administration of rTPO was generally associated with an increase in platelet counts. In some cases it can enhance the megakaryocytes response and therefore cause thrombotic complications. It is suggested that, although thrombocytopenia does not cause platelets to aggregate in the absence of well-known agonists (thrombin, collagen), it does sensitise platelets to the aggregatory effects of these agents. Such "priming" has also been documented in vivo. Platelets derived from thrombocytopenia-treated animals have a heightened sensitivity to substances stimulating platelet aggregation. Thus, TPO may aggrave thrombogenic conditions, and the risk should be carefully evaluated before it is administered to patients. Also, mature platelets remove thrombopoietin from solution, and in thrombocytopenic animals, plasma thrombopoietin concentrations fall soon after platelet transfusion and rise only after the platelet count drops again. This finding that platelets can remove TPO from the circulation has at least two clinical consequences:

i) platelet transfusions may blunt the recovery of megakaryocytes; ii) the binding of TPO to the existing platelets may blunt the response of endogenous TPO to myelosuppressive therapy. However, because the existing platelets continue to bind TPO, the increase in the plasma TPO concentration is delayed until thrombocytopenia intervenes, many days later. In addition, when a truncated form of rTPO was given as a single PEG-conjugated moiety, the therapy was in some cases associated with the development of thrombocytopenia and neutralising antibodies.

In addition to rTPO, several other recombinant cytokines (IL-1, IL-3, IL-6, IL-11, GM-CSF, Steel factor and promegapoietin - IL-3-thrombopoietin fusion protein) have direct and indirect stimulatory effects in vivo and in vitro cells of the megacaryocytic lineage. However, most of these substances have not had beneficial effects on platelet recovery after myelosuppressive therapy or have had unacceptable toxic effects. In contrast to this, IL-11 has proved to be both effective and relatively safe. IL-11 is used to reduce platelet transfusion requirements in cancer patients receiving chemotherapy. When given subcutaneously on a daily basis, IL-11 induces an increase in platelet count in cancer patients. However, the drug has adverse effects primarily due to plasma volume expansion (atrial arrhythmias, palpitations, peripheral edema, headache, dyspnea, anemia, myalgias, weight gain, anorexia, nausea) and in some cancer patients the formation of antibodies (below those considered neutralising) has been observed. IL-11 appears only to be suitable for patients experiencing severe and therapy-limiting thrombocytopenia, or those who have required prior platelet transfusion. It is not recommended for the routine use of attenuating thrombocytopenia.

The DNA sequences and encoded peptide sequences for human TPO-R have also been described (Vigon et al. (1992) Proc. Natl. Acad. Sci. USA 89:5640-5644). TPO-R is a member of the haematopoietin growth factor receptor family, a family characterised by a common structural design of the extracellular domain, including four conserved cysteine residues in the N-terminal portion.

The availability of cloned genes for TPO-R facilitates the search for agonists of this important receptor. The availability of the recombinant receptor protein allows the study of receptor-ligand interaction in a variety of random and semi-random peptide diversity generation systems.

WO 99/42127 discloses a TPO-receptor peptide (in the following termed TPO-Rp wild type) consisting of 23 amino acids which correspond to amino acids 444 to 466 of the human TPO-Rp. A method is disclosed for modulating the activity of TPO-R by applying TPO-Rp to the receptor. However, a specific treatment of thrombocytopenia is not disclosed.

Various authors report on deletion mutants of TPO-R allowing the analysis of structure-function relationships: Dorsch et al. J. Exp. Med. (1997) 186, 1947-1955, Drachman and Kaushansky, Proc. Natl. Acad. Sci. USA (1997) 94, 2350-2355, Porteu et al., Mol. Cell. Biol. (1996), 2473-2482 and Takatoku et al., J. Biol. Chem. (1997) 272, 7259-7263.

Further thrombopoietin receptor agonist peptides are known, for instance from Kimura et al. (J. Biochem. (1997) 122, 1046-1051, Biochem. Mol. Biol. Int. (1998) 44, 1203-1209) disclosing a 15 amino acid peptide from a random phage peptide library which stimulated the proliferation of thrombopoietin dependent cells and the differentiation of mouse bone marrow cell to megakaryocytes. Cwirla et al. disclose a 14 amino acid thrombopoietin receptor agonist peptide stimulating in vitro proliferation and maturation of megakaryocytes from human bone marrow cells (Science (1997) 276, 1696-1699).

There is no disclosure as to whether these deletion mutants of TPO-R or agonist peptides prove useful as mimics of thrombopoietin with the potential to be developed into a stable and pharmaceutically effective drug.

Thus, the technical problem underlying the present invention is to provide an effective compound useful for diagnosing and treating hemotological disorders such as idiopathic and induced thrombocytopenia, in particular chemotherapy, allergic and irradiation induced thrombocytopenia while being non-toxic and stable.

The present invention solves the above problem by providing an isolated and purified oligopeptide with the biological activity of a TPO-R modulator comprising, in particular consisting essentially, preferably consisting, of 15 to 18 amino acids and having the general formula

$$X_1 \: G \: T \: L \: E \: L \: X_2 \: P \: X_3 \: S \: R \: Y \: R \: L \: Q \: L \: X_4,$$

wherein

$X_1$ is A R G or is missing,

$X_2$ is R or A,

$X_3$ is R or A and

$X_4$ is R A R or is missing.

In a particularly preferred embodiment of the present invention, the oligopeptide comprises, in particular consists essentially, preferably consists, of an amino acid sequence as defined in any one of SEQ ID Nos. 1, 2, 3, 4, 5 or 6, which are fully incorporated in the present teaching.

The present invention is inter alia based upon the finding that the above oligopeptides modulate the activity of TPO-R, in particular strongly bind and activate TPO-R and also improve the utilisation of endogenous TPO. The oligopeptides of the present invention therefore show at least two different activities, namely (i) a modulating effect on the TPO-R activity, for instance an agonistic effect such as a TPO-mimic effect or an antagonistic effect and (ii) a synergistic effect together with TPO, in particular when both compounds are present in submaximal concentrations. The synergistic effect is of particular importance as it provides clinical advantages over the use of TPO alone.

Moreover, the oligopeptides of the present invention show a very high potency and efficacy, i.e. are active in the nM to μM range. The oligopeptides of the present invention may be applied alone or in combination with TPO. In contrast to TPO, the oligopeptides of the present invention do not result in down-regulation of the TPO receptor and therefore not in reduced sensitivity to TPO. In contrast, exogenous administration of TPO may result in such receptor down-regulation and thereby reduce sensitivity or tolerance induction. The oligopeptides of the present invention furthermore are very selective for TPO-R and show for instance no cross-reactivity with the closely related erythropoietin receptor.

The present invention furthermore shows that TPO-Rp wild type also has activities useful for specifically treating hematological disorders and in particular thrombocytopenia. Thus, the oligopeptides of the present invention as well as the wild type TPO-Rp appear to be particularly useful for diagnosing and in particular treating hematological disorders, for instance diseases due to platelet disorders and all the various types of thrombocytopenia, either alone or together with TPO or platelets. Due to their small size and high stability in pharmaceutical compositions, the oligopeptides of the present invention are also advantageous insofar as they allow inter alia its oral application. They furthermore show a high degree of stability in vivo.

Without being bound by theory, it appears as if the oligopeptides of the present invention inter alia modulate the TPO-R activities. Namely, binding of TPO results in receptor dimerization and activation of intracellular signalling pathways. In the course of this event, specific phosphorylation of receptor associated kinases of the JAK kinase families (JAK2 and Tyk2) and subsequent phosphorylation and dimerization of the transcription factor STAT5 occurs. Activated STAT5 protein enters the nucleus and binds to the promoter region of target genes stimulating cell proliferation and increasing platelet numbers. However, although the above wild type mechanism of action may be valid also for the present oligopeptides, it cannot be excluded that the present oligopeptides act differently, e.g. via binding to a receptor monomer and thereby mimicking the second chain of the receptor or binding to sites of the receptor, where natural TPO does not bind. In fact, it appears as if the present oligopeptides act on a different site as natural TPO.

The present invention also relates to a Y14F substitution mutant derivate of wild type TPO-Rp, i.e. a wild type TPO-Rp wherein the tyrosin at position 14 of wild type TPO-Rp has been replaced with a phenylalanine. Such a modified peptide may prove particularly valuable for performing e.g. peptide degradation studies since such a peptide may be specifically iodinated at its N- or C-terminal ends.

The oligopeptides of the present invention and the wild type TPO-Rp are useful for the prevention and treatment of diseases mediated by TPO, and particularly for treating hematological disorders, including but not limited to, platelet disorders and thrombocytopenia resulting from allergic reactions, chemotherapy, radiation therapy, or bone marrow transfusions or idiopathic thrombocytopenia. Thus, the present invention also provides a method for treating the above disorder wherein a patient having the disorder that is susceptible to treatment with a TPO-R modulating compound, in particular a TPO agonist receives, or is administered, a therapeutically effective dose or amount of an oligopeptide of the present invention and/or of wild type TPO-Rp.

The invention also provides for pharmaceutical compositions comprising one or more of the oligopeptides or/ and wild type TPO-Rp described herein and a physiologically acceptable carrier. These pharmaceutical compositions can have a variety of forms including oral dosage forms, as well as inhalable powders and solutions and injectable and infusible solutions.

In a particularly preferred embodiment of the present invention, such a composition and method also comprise the use of TPO in combination with the wild type TPO-Rp and/or the oligopeptides of the present invention.

The following definitions are set forth to illustrate and define the meaning and scope of the various terms used to describe the present invention.

The terms "transformation" or "transfection" mean the act of causing a host cell to contain a desired nucleic acid molecule, including either a native TPO-Rp nucleotide sequence or a nucleotide sequence encoding the present oligopeptides not originally part of that cell or not in the natural location, copy number or orientation using methods known in the art.

By "operably linked" it is meant that a gene and at least one regulatory sequence are connected in sense or antisense expression in such a way as to permit gene expression when the appropriate molecules (e.g. transcriptional activator proteins) are bound to the regulatory sequence.

The term "vector" refers to a recombinant DNA construct which may be a plasmid, virus, or autonomously replicating sequence, phage or nucleotide sequence, linear or circular, of a single or double stranded DNA or RNA, derived from any source, in which a number of nucleotide sequences have been joined or recombined into a unique construction which is capable of introducing a promoter fragment and DNA sequence for a selected gene product in sense or antisense orientation along with an appropriate 3' untranslated sequence into a cell.

"Plasmids" are genetic elements that are stably inherited without being a part of the chromosome of their host cell. They may be comprised of DNA or RNA and may be linear or circular. Plasmids code for molecules that ensure their replication and stable inheritance during cell replication and may encode products of considerable medical, agricultural and environmental importance. They can also encode genes that confer resistance to antibiotics. Plasmids are widely used in molecular biology as vectors to clone and express recombinant genes. Starting plasmids disclosed herein are either commercially available, publicly available, or can be constructed from available plasmids by routine application of well known, published procedures. Many plasmids and other cloning and expression vectors that can be used in accordance with the present invention are well known and readily available to those of skill in the art. Moreover, those of skill may readily construct any number of other plasmids suitable for use in the invention. The properties, construction and use of such plasmids, as well as other vectors, in the present invention will be readily apparent to those of skill from the present disclosure.

The term "host cell" refers to a cell which has been genetically modified by transfer of a chimeric, heterologous or autologous nucleic acid sequence or its descendants still containing this sequence. These cells are also termed "transgenic cells". In the case of an autologous nucleic acid sequence being transferred, the sequence will be present in the host cell in a higher copy number, in another genetic environment or another orientation than naturally occurring.

By "solid support" an insoluble matrix is meant, either biological in nature, such as, without limitation, a cell or bacteriophage particle, or synthetic, such as, without limitation, an acrylamide derivate, cellulose, nylon, silica and magnetised particles, to which soluble molecules may be linked or joined.

The term "antibody" refers to a polypeptide substantially encoded by an immunoglobulin gene or immunoglobulin genes, or fragments thereof, which specifically bind and recognise an analyte (antigen). The recognised immunoglobulin genes include the kappa, lambda, alpha, gamma, delta, epsilon and mu constant region genes, as well as the myriad immunoglobulin variable region genes. Antibodies exist, e.g., as intact immunoglobulins or as a number of well characterised fragments produced by digestion with various peptidases. "Antibody" also refers to modified antibodies (e.g. oligomeric, reduced, oxidated and labelled antibodies). The term "antibody", as used herein, also includes antibody fragments either produced by the modification of whole antibodies or those synthesised de novo using recombinant DNA methodologies. The term "antibody" includes intact molecules as well as fragments thereof, such as Fab, $F(ab')_2$, and Fv which are capable of binding the epitopic determinant. These antibody fragments retain some ability to selectively bind with its antigen or receptor and are defined as follows:

1) Fab, the fragment which contains a monovalent antigen-binding fragment of an antibody molecule can be produced by digestion of whole antibody with the enzyme papain to yield an intact light chain and a portion of one heavy chain;

(2) Fab', the fragment of an antibody molecule can be obtained by treating whole antibody with pepsin, followed by reduction, to yield an intact light chain and a portion of the heavy chain; two Fab' fragments are obtained per antibody molecule;

(3) $(Fab')_2$, the fragment of the antibody that can be obtained by treating whole antibody with the enzyme pepsin without subsequent reduction; $F(ab')_2$ is a dimer of two Fab' fragments held together by two disulfide bonds;

(4) Fv, defined as a genetically engineered fragment containing the variable region of the light chain and the variable region of the heavy chain expressed as two chains; and

(5) Single chain antibody ("SCA"), defined as a genetically engineered molecule containing the variable region of the light chain, the variable region of the heavy chain, linked by a suitable polypeptide linker as a genetically fused single chain molecule.

Methods of making these fragments are known in the art. (See for example, Harlow and Lane, Antibodies: A Laboratory Manual, Cold Spring Harbor Laboratory, New York (1988)).

As used in this invention, the term "epitope" means any antigenic determinant on an antigen to which the paratope of an antibody binds. Epitopic determinants usually consist of chemically active surface groupings of molecules such as amino acids or sugar side chains and usually have specific three dimensional structural characteristics, as well as specific charge characteristics.

Monoclonal antibodies to the proteins of the present invention, and to the fragments thereof, can also be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. See, e.g., M. Schreier et al., "Hybridoma Techniques" (1980); Hammerling et al., "Monoclonal Antibodies and T-cell Hybridomas" (1981); Kennett et al., "Monoclonal Antibodies" (1980); see also U.S. Pat. Nos. 4,341,761; 4,399,121; 4,427,783; 4,444,887; 4,452,570; 4,466,917; 4,472,500; 4,491,632; and 4,493,890. Panels of monoclonal antibodies produced against the protein of interest, in particular TPO-Rp or the present oligopeptides or fragments thereof, can be screened for various properties; i.e., for isotype, epitope, affinity, etc. Alternatively, genes encoding the monoclonals of interest may be isolated from the hybridomas by PCR techniques known in the art and cloned and expressed in the appropriate vectors. Monoclonal antibodies are useful in purification, using immunoaffinity techniques of the individual proteins against which they are directed. The antibodies of this invention, whether polyclonal or monoclonal, have additional utility in that they may be employed as reagents in immunoassays, RIA, ELISA, and the like. In addition, they can be used to detect and/or isolate the present oligopeptides from cell extracts or cells. The antibodies e.g. could be used to establish a tissue culture based assay for discovery or modification of novel compounds which modulate TPO-R activity, for instance mimic TPO activity.

The humanised or chimeric antibodies can comprise portions derived from two different species (e.g., human constant region and murine binding region). The portions derived from two different species can be joined together chemically by conventional techniques or can be prepared as a single fusion protein using genetic engineering techniques. DNA encoding the proteins of both portions of the chimeric antibody can be expressed as a single fusion protein.

An antibody "specifically binds to" or "is specifically immunoreactive with" a protein when the antibody functions in a binding reaction which is determinative of the presence of the protein in the presence of a heterogeneous population of proteins and other biologics. Thus, under designated immunoassay conditions, the specified antibodies bind preferentially to a particular protein and do not bind in a significant amount to other proteins present in the sample. Specific binding to a protein under such conditions requires an antibody that is selected for its specificity for a particular protein. A variety of immunoassay formats may be used to select antibodies specifically immunoreactive with a particular protein. For example, solid-phase ELISA immunoassays are routinely used to select monoclonal antibodies specifically immunoreactive with a protein. See Harlow and Lane (1988) Antibodies, A Laboratory Manual, Cold Spring Harbor Publications, New York, for a description of immunoassay formats and conditions that can be used to determine specific immunoreactivity.

The term "immunoassay" refers to an assay that utilises an antibody or antigen to specifically bind an analyte, which can also be an antibody or an antigene. The immunoassay is characterised by the use of specific binding properties of a particular antibody to detect-, isolate, target, and/or quantify the analyte. Either the antibody or the antigen may be labelled to allow detection.

In the context of the present invention, the term "treatment" refers to a prophylactic and/or therapeutic effect of a drug or medicament which in turn is defined as a composition comprising a pharmaceutically or diagnostically effective compound in combination with at least one additive, such as a carrier.

"Agonist" refers to a biologically active ligand which binds to its complementary biologically active receptor and activates the latter either to cause a biological response in the receptor or to enhance pre-existing biological activity of the receptor. A TPO agonist binds to the TPO receptor (TPO-R). The TPO agonist may act as a TPO mimic. However, the TPO agonist may also activate or contribute to the activation of TPO-R by the use of binding sites or mechanisms different from those for TPO.

"Antagonist" refers to a biologically active ligand which binds to its complementary biologically active receptor and suppresses, reduces or abolishes the activity of TPO-R, for instance by the use of binding sites or mechanisms different or similar from those for TPO.

The term "pharmaceutically acceptable salts" refers to the non-toxic alkali metal, alkaline earth metal, and ammonium salts commonly used including the ammonium, barium, calcium, lithium, magnesium, potassium, protamine zinc salts and sodium, which are prepared by methods known in the art. The term also includes non-toxic; i.e. pharmaceutically acceptable acid addition salts, which are generally prepared by reacting the oligopeptides of the present invention with a suitable organic or inorganic acid, such as acetate, benzoate, bisulfate, borate, citrate, fumarate, hydrobromide, hydrochloride, lactate, laurate, maleate, napsylate, oleate, oxalate, phosphate, succinate, sulfate, tartrate, tosylate, valerate, etc.

The term "pharmaceutically acceptable acid addition salt" refers to salts which retain the biological effectiveness and properties of the free bases and which are not biologically or otherwise undesirable, formed with inorganic acids such as hydrobromic acid, hydrochloric acid, nitric acid, phosphoric acid, sulfuric acid, and organic acids such as acetic acid, benzoic acid, cinnamic acid, citric acid, ethanesulfonic acid, fumaric acid, glycolic acid, maleic acid, malic acid, malonic acid, mandelic acid, menthanesulfonic acid, oxalic acid, propionic acid, p-toluenesulfonic acid, pyruvic acid, salicylic acid, succinic acid, tartaric acid, etc.

The term "pharmaceutically acceptable ester" refers to esters which retain, upon hydrolysis of the ester bond, the biological effectiveness and properties of its constituents, namely the carboxylic acid or alcohol and are not biologically or otherwise undesirable. The present invention also contemplates the use of those compositions which are both esters as described above and at the same time are the pharmaceutically acceptable acid addition salts thereof.

The salts of the invention can be obtained by dissolving the free oligopeptide in an aqueous or aqueous/alcoholic solvent or in other suitable solvents with an appropriate base and then isolating the obtained salt of the invention by evaporating the solution, by freezing and lyophilization or by addition of another solvent, e.g. diethylether, to the aqueous and/or alcoholic solution of the oligopeptide salt including the separation of unsoluble crued salt. For salt formation, usually one or maximally two mols of base, i.e. cation, and one mol of the free oligopeptide are used. For the preparation of alkali oligopeptide salts, alkali metal carbonates or hydrogencarbonates are preferably used. The prepared peptide salts are freely soluble in water. Thus, the present invention also relates to a process for the preparation of the oligopeptide salts.

In the context of the present invention, a base is considered as a substance capable of forming a cation in a solution, particularly in an aqueous and aqueous/alcoholic solution.

The term "pharmaceutically acceptable amide" refers to amides which retain, upon hydrolysis of the amide bond, the biological effectiveness and properties of the carboxylic acid or amine and are not biologically or otherwise undesirable. These amides are typically formed from the corresponding carboxylic acid and an amine. This invention also contemplates the use of those compositions which are both amides as described and at the same time are the pharmaceutically acceptable acid addition salts thereof.

Techniques for the preparation of pharmaceutically acceptable esters and amides are for instance disclosed in March Advanced Organic Chemistry, 3rd Ed., John Wiley & Sons, New York (1985) p. 1152. Pharmaceutically acceptable esters and amides useful as prodrugs are disclosed in Bundgaard, H., ed., (1985) Design of Prodrugs, Elsevier Science Publishers, Amsterdam.

The term "pharmaceutically or therapeutically acceptable carrier" refers to a carrier medium which does not interfere with the effectiveness of the biological activity of the active ingredients and which is not toxic to the host or patient.

"Therapeutically- or pharmaceutically-effective amount" as applied to the oligopeptides and compositions of the present invention refers to the amount of oligopeptide or composition sufficient to induce a desired biological result. That result can be alleviation of the signs, symptoms, or causes of a disease, or any other desired alteration of a biological system. In the present invention, the result will for instance in a particularly preferred embodiment involve a TPO mimic activity, namely preventing, abolishing and/or reducing selectively thrombocytopenia symptoms, for instance raising platelet counts, and/or preventing platelet count drops.

Amino acid residues in the present oligopeptides are abbreviated as conventionally done as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic acid is Asp or D; Glutamic acid is Glu or E; Cysteine is Cys or C; Tryptophan is Trp or W; Arginine is Arg or R; and Glycine is Gly or G.

Thus, for instance A R G is a continuous stretch, i.e. a tripeptide consisting of Alanine, Arginine and Glycine while R A R is a continuous stretch of Arginine, Alanine and Arginine.

The present invention not only relates to the oligopeptides specifically mentioned in SEQ ID No. 1 to 6 but also to biological equivalents, i.e. substances having different structures but displaying similar or comparable biological effects, in particular derivatives thereof which have similar or comparable structures and/or functions and act as TPO-R modulator. These biological equivalents, in particular derivates may differ from the oligopeptides of the present invention with respect to susceptibility to hydrolysis or proteolysis and/or with respect to other biological properties, such as increased affinity for the TPO receptor. Thus, the invention also relates for instance to pharmaceutically acceptable salts, amides or esters of the oligopeptides of the present invention.

Thus, in addition to oligopeptides consisting only of naturally-occurring amino acids, peptidomimetics or peptide analogs are also provided. Peptide analogs are commonly used as non-peptide drugs with properties analogous to those of the template peptide. These types of non-peptide compounds are termed "peptide mimetics" or "peptidomimetics". Peptide mimetics that are structurally similar to therapeutically useful peptides may be used to produce an equivalent or enhanced therapeutic or prophylactic effect. Generally, peptidomimetics are structurally similar to a paradigm polypeptide (i.e., a polypeptide that has a biological or pharmacological activity), such as naturally-occurring receptor-binding polypeptide, but have one or more peptide linkages optionally replaced by a linkage selected from the group consisting of: $-CH_2-NH-NH-$, $-C-CH_2-S-$, $-CH_2-CH_2-$, $-CH=CH-$ (cis and trans), $-COCH_2-$, $-CH(OH)-CH_2-$, and $-CH_2-SO-$, by methods known in the art. A particularly preferred non-peptide linkage is $-CH_2-NH-$. Such peptide mimetics may have significant advantages over polypeptide embodiments including, for example: improved chemical stability, enhanced pharmacological properties (half-life, absorption, potency, efficacy, etc.), more economical production altered specificity (e.g., a broad-spectrum of biological activities), reduced antigenicity, etc. Labelling of peptidomimetics usually involves covalent attachment of one or more labels, directly or through a spacer e.g., an amide group, to non-interfering positions on the peptidomimetic that are predicted by quantitative structure-activity data and/or molecular modelling. Such non-interfering positions generally are positions that do not form direct contacts with the macromolecules(s) e.g., immunoglobulin superfamily molecules to which the peptidomimetic binds to produce the therapeutic effect. Derivatisation e.g., labelling of peptidomimetics should not substantially interfere with the desired biological or pharmacological activity of the peptidomimetic. Generally, peptidomimetics of receptor-binding peptides bind to the receptor with high affinity and possess detectable biological activity, i.e. are agonistic or antagonistic to one or more receptor-mediated phenotypic changes.

In the context of the present invention, substitution of one or more L-amino acids with a D-amino acid of the same type (e.g., D-lysine in place of L-lysine) may be used to generate more stable peptides.

In addition, the present invention relates to oligopeptides comprising the consensus sequence identified by the above general formula or a substantially identical consensus sequence variation which may be generated by methods known in the art for example, by adding internal cysteine residues capable of forming intramolecular disulfide bridges which cyclize the peptide.

The present invention not only relates to the above oligopeptides in linearized form but of course also relates to cyclized oligopeptides, for instance cyclized by an amide bond between the first and last amino acid.

"Synthetic or non-naturally occurring amino acids" refer to amino acids which do not naturally occur in vivo but which, nevertheless, can be incorporated into the oligopeptide of the present invention. Other preferred synthetic amino acids include amino acids wherein the amino group is separated from the carboxyl group by more than one carbon atom such as β-alanine or γ-aminobutyric acid. Particularly preferred synthetic amino acids include the D-amino acids of naturally occurring L-amino acids, L-1-napthyl-alanine, L-2-naphthylalanine, L-cyclohexylalanine, L-2-amino isobutyric acid, the sulfoxide and sulfone derivatives of methionine.

"Detectable label" refers to substances, which when covalently attached to the oligopeptides, oligopeptide mimetics and/or antibodies of the present invention, permit detection of the oligopeptide and oligopeptide mimetics in vivo in the system, for instance the patient to whom the oligopeptide or oligopeptide mimetic has been administered or in vitro. Suitable detectable labels are well known in the art and include, by way of example, radioisotopes and fluorescent labels (e.g., fluorescein).

Covalent attachment of the detectable label to the oligopeptide or oligopeptide mimetic is accomplished by conventional methods well known in the art. For example, when the 125 I radioisotope is employed as the detectable label, covalent attachment of 125 I to the oligopeptide or the oligopeptide mimetic can be achieved by incorporating the amino acid tyrosine into the oligopeptide or oligopeptide mimetic and then iodating the oligopeptide. Also, 32 P can be incorporated onto the oligopeptide or oligopeptide mimetic as a phosphate moiety through, for example, a hydroxyl group on the peptide or peptide mimetic.

The oligopeptides of the invention may be prepared by conventional methods known in the art, for example, by using standard solid phase techniques. The standard methods include, but are not limited to, exclusive solid phase synthesis, partial solid phase synthesis methods, fragment condensation, classical solution synthesis, and by recombinant DNA technology. The oligopeptides of the present invention may thus be prepared directly by recombinant methods (see Sambrook et al. Molecular Cloning: A Laboratory Manual, CSHL Press, Cold Spring Harbor, NY 1989) or as a fusion protein, for example to a protein that is one of a specific binding pair, allowing purification of the fusion protein by means of affinity reagents, followed by proteolytic cleavage, usually at a site engineered to yield the desired peptide.

The oligopeptides may be extended to provide convenient linking sites, e.g., cysteine or lysine, to enhance stability, to bind to particular receptors, to provide for site-directed action, to provide for ease of purification, to alter the physical characteristics (e.g. solubility, charge, etc.) to stabilise the conformation, etc. The oligopeptides may be joined to non-wild-type flanking regions as fused proteins, joined either by linking groups or covalently linked through cysteine (disulfide) or peptide linkages. The oligopeptide may be linked through a variety of bifunctional agents, such as maleimidobenzoic acid, methyldithioacetic acid, mercaptobenzoic acid, S-pyridyl dithiopropionate, etc. The oligopeptides may be joined to a single amino acid at the N- or C-terminus of a chain of amino acids, or may be internally joined. For example, the present oligopeptides may be covalently linked to an immunogenic protein, such as keyhole limpit hemacyanin, ovalbumin, etc. to facilitate antibody production to the subject oligopeptides.

The oligopeptides of the present invention may be expressed in conjunction with other peptides or proteins, so as to be a portion of the chain, either internal or at the N- or C-terminus. Such a fused oligopeptide is called a fusion or conjugate peptide. Various post-expression modifications may be achieved, including glycosylations. For example, by employing the appropriate coding sequences, one may provide farnesylation or prenylation, such that the subject peptide will be bound to a lipid group at one terminus, and will be able to be inserted into a lipid membrane, such as a liposome. The oligopeptides of the present invention may be PEGylated, where the polyethylenoxy group provides for enhanced lifetime in the blood stream. The oligopeptides of the present invention may also be associated to serum protein for instance albumins. The oligopeptides may also be combined either by protein fusion or by association with or to other proteins, such as the Fc of an IgG isotype to enhance complement binding or with a toxin, such as ricin, abrin, diphtheria toxin, or the like, particularly the A chain. The oligopeptides may be linked to antibodies for site directed action. The present invention therefore also provides conjugate peptides comprising i.a. the oligopeptides of the present invention.

The oligopeptides of the present invention may serve as structural models for non-peptidic compounds with similar biological activity. Those of skill in the art recognise that a variety of techniques are available for constructing compounds with the same or similar desired biological activity as the present oligopeptides but with more favourable activity than the lead with respect to solubility, stability, and susceptibility to hydrolysis and proteolysis. These techniques include replacing the peptide backbone with a backbone composed of phosphates, amides, carbonates, sulphonamides, secondary amines, and N-methylamino acids.

Thus, the present invention also relates to recombinantly producing the oligopeptides of the present invention.

Accordingly, the present invention relates to nucleotide sequences encoding the oligopeptides identified in SEQ ID Nos. 1 to 7 which encompass due to the degeneracy of the genetic code more than six various nucleotide sequences. Of course the present invention also relates to the above nucleotide sequences which are mutated for instance by nucleotide additions, deletions, insertions or inversions as long as the encoded oligopeptide has the desired TPO-R modulator effect of the present invention.

The present invention also relates to vectors comprising the above nucleotide sequences which can be introduced and expressed in a host cell system using conventional materials and techniques. DNA elements such as promoters, enhancers, polyadenylation sites, transcription termination signals, etc. should be associated with the nucleotide sequences so as to promote and control expression. The specific regulatory element used will depend upon the host cell system selected for expression whether secretion of the oligopeptide or the conjugate peptide is desired. The vector may be in a preferred embodiment of the present invention a bacterial, viral, mammalian or yeast vector, which in a particularly preferred embodiment comprises the above identified 5' and/or 3' regulatory elements capable of directing expression of the nucleotide sequence in a suitable host cell.

Various vectors may be employed as vehicles for the introduction and expression of the present oligopeptides in a host cell. Such vectors useful in the different host cell types are well known and include, for example, the mammalian expression vectors pSG5 (Stratagene), p-RK1 (Genetics Institute), p-SVK3 (Pharmacia), p-EUK-C1 (Clontech), pCDM (Invitrogen), pc DNAI (Invitrogen), and the bacterial expression vectors pFLAG-1 (IBI), all pET system plasmids (Novagen), pTrcHis (Invitrogen), the pGEX series (Pharmacia) and pKK 233-2 (Clontech). These vectors may be maintained as episomes in the host cell or they may facilitate integration of the present nucleotide sequences into the host cell genome, or both. Vectors may also include other useful features, such as genes which allow for the selection or detection of cells in which they have been successfully introduced.

Host cells suitable for expression of the nucleotide sequences of the present invention include, but are not limited to procaryotic and eucaryotic hosts, such as Bacillus subtilis, E. coli, yeast, Xenopus laevis oocytes, insect cells, plant cells, and a variety of mammalian cell types, including in particular Chinese Hamster Ovary (CHO) cells, Hela-cells, L(tk-) cells, primary cultures, Cos17 cells, Cos1 cells, baby hamster kidney cells and CV1 cells.

Host cells which provide for glycosylation are also included in the present invention.

The present invention also relates to methods for genetically modifying a cell by transfecting the cell with the above identified vector. The transfection maybe achieved by conventional methods such as biological, physical, chemical or electrical induced transfection, in particular electroporation, cell fusion, retrovirus or virus mediated gene transfer, lipsome mediated gene transfer or particle bombardment.

The present invention also relates to methods for producing non-human mammalian animals capable of producing the oligopeptides of the present invention in their cells wherein a nucleotide sequence of the present invention is introduced into a non-human mammalian animal cell, in particular not later than in the 8-cell-, preferably the 1-cell-stage which subsequently is cultivated under appropriate conditions so as to obtain an adult differentiated animal. Such an animal might comprise in its germ cells or somatic cells, in particular in its chromosome a nucleotide sequence of the present invention capable of expressing the oligopeptide of the present invention. In a particularly preferred embodiment of the present invention the mammalian animal is a rodent or primate. Such a method may allow various kinds of gene therapy, e.g. somatic gene therapy or germ line gene therapy.

The present invention also encompasses genetically manipulated, in particular transgenic animals, especially mammals, in particular primates and mice and cells thereof. These animals, containing in at least some of their cells for instance transfected sense or antisense constructs of the nucleotide sequences of the present invention under control of regulatory elements are useful for research and diagnosis purposes because the activity of TPO-R is modified. The modification of TPO-R in transgenic animals is possible e.g. by using sense or antisense nucleotide sequences of the present invention, or any modifications of these nucleotide sequences such as inversions, deletions, insertions, additions, etc. to transform and obtain such animals being genetically manipulated. In one embodiment of the present, vectors or oligonucleotides of the present invention are transfected and integrated in the genome of the non-human mammalian cell, so as to express an oligopeptide capable of modulating TPO-R activity. In another embodiment of the present invention vectors or oligonucleotides of the present invention are transfected and inserted in the genome, in particular in the endogenous TPO-R gene by homologous recombination such as to produce animals expressing a modified TPO-R. Thus, the present invention also relates to animals being genetically modified, in particular being transgenic animals which exhibit a modified TPO-R function, in contrast to the wild-type animal. Such a modified function in a mammalian, in particular a non-human mammalian cell may be due to the introduction of antisense or sense constructs of the present invention, possibly containing nucleotide sequence alterations and/or may be due to manipulations in the endogenous nucleotide sequences for TPO-R. By virtue of these modifications, such as insertions of additional mutated or non-mutated sense or antisense copies of the TPO-Rp coding sequences designed in accordance with the present invention or modifications in the endogenous genes, it is possible to obtain useful animals for the above-identified purposes. The present invention thus also relates to single non-human mammalian cells or cell cultures containing the above identified modifications.

The present invention also relates to a method for the preparation of an oligopeptide of the present invention comprising transfecting a cell with the vector according to the present teaching, culturing the cell in a culture medium under conditions allowing the expression of the oligopeptide and recovering the oligopeptide from the cell or culture medium using conventional techniques.

The present invention also relates to monoclonal or polyclonal antibodies or fragments thereof specifically binding to the present oligopeptides. These antibodies may be used to detect and isolate the present oligopeptides, structural analogs thereof or even TPO-R itself. In case the oligopeptides or TPO-R are present in TPO-R containing or oligopeptide containing sources such as a cell, cell part or a cell organelle, further manipulation prior to detecting or isolating may be necessary, such as conventional methods to disrupt biological material, e.g. enzymatic cell lysis.

The invention also relates to monoclonal or polyclonal antibodies specifically recognising and binding to the above antibodies.

The present invention also relates to an immunoassay for detecting and/or isolating the oligopeptides of the present invention from a mixture containing the cligopeptides wherein the antibodies of the present invention are applied to the mixture and the oligopeptides are detected and /or isolated. Vice versa, the immunoassay may be used to detect the antibodies of the present invention by using the oligopeptides of the present invention as probe.

The oligopeptides of the present invention are useful in vitro as unique tools for analysing the biological role of TPO, including the evaluation of the many factors involved in the production of TPO and the receptor binding process. The oligopeptides of the present invention are also useful in the development of other compounds that bind to and activate TPO-R, because the present oligopeptides provide important information on the relationship between structure and activity.

The oligopeptides of the present invention are also useful as competitive binders in assays to screen for further TPO receptor agonists. The oligopeptides of the invention can be used without modification or may be modified such as covalently or non-covalently joining a labelling moiety which directly or indirectly provides a detectable signal. Direct labelling includes label groups such as: radiolabels, enzymes such as peroxidase and alkaline phosphatase, and fluorescent labels capable of monitoring the change in fluorescence intensity, wavelength shift, or fluorescence polarisation. Indirect labelling includes biotinylation of one constituent followed by binding to avidin coupled to one of the above label groups. The compounds may also include spacers in cases where the compounds are to be attached to a solid support.

Based on their ability to bind to the TPO receptor, the oligopeptides of the present invention can be used as reagents for detecting TPO receptors on membranes, cell organs, compartments, living cells, fixed cells, in biological fluids, in tissue homogenates, in purified, natural biological materials, in crude extracts, etc. For example, by labelling the present oligopeptides, one can identify cells having TPO-R on their surfaces. Furthermore, based on their ability to bind TPO-R, the oligopeptides of the present invention can be used in in situ staining, FACS (fluorescence-activated cell sorting), Western blotting, ELISA, etc. In addition, based on their ability to bind to TPO-R, the oligopeptides of the present invention can be used in methods for TPO-R isolation and purification, or in isolation and purifying cells expressing TPO-R on the cell surface or inside permeabilized cells.

The present invention also relates to the use of the oligopeptides of the present invention which are immobilised, for instance on a solid support, according to conventional methods, for the above screening and isolation procedures. In one embodiment of the present invention, in particular of the present screening assay, the oligopeptide is non-diffusably bound to an insoluble support having isolated sample receiving areas, e.g. a micortiter plate. The insoluble supports may be made of any composition to which the oligopeptide or receptor can be bound, is readily separated from soluble material, and is otherwise compatible with the overall method of screening. The surface of such supports may be solid or porous and of any convenient shape. Examples of suitable insoluble supports include microtiter plates, membranes and beads. These are typically made of glass, plastic e.g. polystyrene, polysaccharides, nylon or nitrocellulose.

Of course, the present invention also relates to the above isolation and screening methods using the oligopeptides and/or antibodies against these oligopeptides in high throughput screening and/or isolation methods, for instance in solution without using immobilized agents.

The oligopeptides of the present invention can also be utilised as commercial reagents for various medical research and diagnostic uses. Such uses include but are not limited to: (1) use as a calibration standard for quantifying the activities of TPO or potential TPO agonists in various functional assays; (2) use to maintain the proliferation and growth of TPO-dependent cell lines; (3) use in structural analysis of the TPO-receptor through co-crystallization; (4) use to investigate the mechanism of TPO signal transduction/receptor activation; and (5) other research and diagnostic applications wherein the TPO-receptor is preferably activated or such activation is conveniently calibrated against a known quantity of TPO or a TPO agonist.

The oligopeptides of the present invention may be used for the in vitro expansion of megakaryocytes and their committed progenitors, both together with additional cytokines, such as TPO or alone. Chemotherapy and irradiation cause thrombocytopenia by killing the rapidly dividing, more mature population of megakaryocytes. However, these therapeutic treatments can also reduce the number and viability of the immature, less mitotically active megakaryocyte precursor cells. Accordingly, amelioration of the thrombocytopenia by the oligopeptides of the present invention may in one embodiment of the present invention be improved by using patients after finalising the chemotherapy or radiation therapy with a population of the patient's own cells enriched for megakaryocytes and immature precursors by in vitro culture.

The oligopeptides of the invention and/or wild type TPO-Rp and/or antibodies specifically binding thereto can also be administered to animals, including mammals such as rodents and primates, including humans, to modulate, in particular activate TPO-R in vivo and/or to increase or maintain the existing platelet count. Thus, the present invention encompasses methods for therapeutic treatment of TPO related disorders that comprise administering an oligopeptide of the invention in amounts sufficient to modulate the effect of TPO-R in vivo. For example, the oligopeptides and compositions of the present invention can be administered to treat a variety of hematological disorders, including but not limited to platelet disorders and thrombocytopenia, particularly when associated with bone marrow transfusions, radiation therapy, and chemotherapy. Such an administration may also include application of TPO.

Thus, the present invention also relates to methods for modulating in particular increasing or decreasing the activity of TPO-R wherein the oligopeptide of the present invention or an antibody specifically binding thereto is applied to TPO-R either in the absence or in the presence of TPO. Such a method may be an in vivo or an vitro method.

The oligopeptides and compositions of the present invention will in a preferred embodiment be administered prophylactically prior to or simultaneously with chemotherapy, radiation therapy, or bone marrow transplant or after such exposure.

Accordingly, the present invention also provides pharmaceutical compositions comprising, as an active ingredient, at least one of the oligopeptides of the invention and/or TPO-Rp wild type oligopeptide in association with a pharmaceutical carrier or diluent. The compositions of this invention can be administered systematically or topically, in particular by intravascular oral, pulmonary, parental, e.g. intramuscular, intraperitoneal, intravenous (IV) or subcutaneous injection or inhalation, e.g. via a fine powder formulation, transdermal, nasal, vaginal, rectal, or sublingual routes of administration and can be formulated in dosage forms appropriate for each route of administration. The oligopeptides of the present invention and/or TPO-Rp wild type oligopeptide may be used in such compositions in the form of a pharmaceutically acceptable salt, addition salt, ester, amide, or/and a free base, preferably in a pharmaceutically effective amount.

Solid dosage forms for oral administration include capsules, lingualettes, tablets, pills, powders, liposomes, patches, time delayed coatings and granules. In such solid dosage forms, the active compound is admixed with at least one inert pharmaceutically acceptable carrier such as lactose, sucrose, or starch. Such dosage forms can also comprise additional substances other than inert diluents, e.g., lubricating agents such as magnesium stearate. In the case of capsules, tablets, and pills, the dosage forms may also comprise bulking and/or buffering as well as flavouring agents. Tablets and pills can additionally be prepared with enteric coatings.

Liquid dosage forms for oral administration include pharmaceutically acceptable emulsions, solutions, suspensions, syrups, with the elixers containing inert diluents commonly used in the art, such as water. Besides such inert diluents, compositions can also include adjuvants, such as salts for varying the osmotic pressure, pH-adjusting compounds, skin penetration agents, wetting agents, emulsifying and suspending agents, and sweetening, flavouring, and perfuming agents.

Pharmaceutical compositions according to the present invention for parental administration include sterile aqueous or non-aqueous solutions, suspensions, or emulsions. Examples of non-aqueous solvents or vehicles are propylene glycol, polyethylene glycol, vegetable oils, such as olive oil and corn oil, gelatine, and injectable organic esters such as ethyl oleate. Such dosage forms may also contain adjuvants such as preserving, wetting, emulsifying, and dispersing agents. They may be sterilised by, for example, filtration through a bacteria retaining filter, by incorporating sterilising agents into the compositions, by irradiating the compositions, or by heating the compositions. They can also be manufactured using sterile water, or some other sterile injectable medium, immediately before use.

Formulations for injection will comprise a physiologically-acceptable medium, such as water, saline, PBS, aqueous ethanol, aqueous ethylene glycols and the like. Water soluble preservatives which may be employed include sodium bisulfite, sodium thiosulfate, ascorbate, benzalkonium chloride, chlorobutanol, thimerosal, phenylmercuric borate, parabens, benzyl alcohol and phenylethanol. These agents may be present in individual amounts of from about 0.001 to about 5% by weight and preferably about 0.01 to about 2%. Suitable water soluble buffering agents that may be employed are alkali or alkaline earth carbonates, phosphates, bicarbonates, citrates, borates, acetates, succinates and the like, such as sodium phosphate, citrate, borate, acetate, bicarbonate and carbonate. Additives such as carbomethylcellulose may be used as a carrier in amounts of from about 0.01 to about 5% by weight. The formulation will vary depending upon the purpose of the formulation, the particular mode employed for modulating the receptor activity, the intended treatment, etc.

Compositions for rectal or vaginal administration are preferably suppositories which may contain, in addition to the active substance, excipients such as cocoa butter or a suppository wax. Compositions for nasal or sublingual administration are also prepared with standard excipients well known in the art.

The compositions containing the oligopeptides of the present invention and/or wild type TPO-Rp can be administered for prophylactic and/or therapeutic treatments. In therapeutic applications, compositions are administered to a patient already suffering from a disease, as described above, in an amount sufficient to cure or at least partially arrest the symptoms of the disease and its complications, i.e. a therapeutically effective amount.

In prophylactic applications, compositions containing the oligopeptides of the present invention and/or wild type TPO-Rp are administered to a patient susceptible to or otherwise at risk of a particular disease. Such an amount is defined to be a "prophylactically effective dose." In this use, the precise amounts again depend upon the patient's state of health and weight.

The pharmaceutical compositions of the present invention may also be administered in the form of a depot, such as a slow release composition. Such a slow release composition may include oligopeptide-containing particles in a matrix, made e.g. from collagen.

The quantities of the present TPO agonist necessary for effective therapy will depend upon many different factors, including means of administration, target site, physiological state of the patient, and other medicants administered.

The oligopeptides of the present invention and/or wild type TPO-Rp are effective in treating TPO mediated conditions when administered at a dosage range of from about 0.03 mg to about 10 mg/kg of body weight of the mammal per day, in particular of from about 0.3 to about 1 mg/kg. The specific dose employed is regulated by the particular condition being treated, the route of administration, as well as by the judgement of the attending clinician depending upon factors such as the severity of the condition, and the age and general condition of the patient.

The oligopeptide of the present invention and/or wild type TPO-Rp may be administered alone or together with TPO, the dose of the latter possibly being reduced by 50% or 25% (in contrast to the regular dose of TPO application) due to the TPO enhancement effect of the present oligopeptides.

The composition, preferably the water-soluble composition, of the invention may further contain a water-soluble protein injectable into body fluids without showing any substantial pharmacological activity at the concentration used in one unit dosage form of the present invention (hereinafter, "water-soluble protein"). As such a water-soluble protein, serum albumin, globulin, collagen and/or gelatine are preferred. This protein can be added in an amount generally employed in injectable pharmaceutical compositions. Thus, for example, the weight ratio between the water-soluble protein and the oligopeptide of the present invention is about 0.0001:1 to 100:1, preferably about 0.001:1 to about 10:1 or more preferably about 0.01:1 to about 1:1.

Continuing, the invention also relates to the aforementioned oligopeptides themselves and compositions containing them, in particular, in dried and/or pure form or in an aqueous or aqueous/alcoholic solution. The pH of a solution prepared from the water-soluble composition or a peptide salt of the present invention should be such that said pH will not exert any adverse influence upon the activity of the pharmacologically active peptide, but is within an acceptable range for injections in general and further, such that said pH will neither cause a great change in viscosity of the solution nor allow formation of a precipitate or the like. Thus the solution should preferably have a pH of about 4 to 7, preferably 5 to 6, in particular 5.3 to 5.5.

When the water-soluble composition of the invention is converted into an aqueous solution for administration, the concentration of the pharmacologically active oligopeptide or salt thereof in said solution should preferably be about 0.0000001 to 10 % (w/v), more preferably about 0.000001 to 5% (w/v) or most preferably about 0.00001 to 1% (w/v).

The composition of the present invention should preferably have a unit dosage form containing the pharmacologically active oligopeptide of the invention and, if necessary, together with further additives such as the above mentioned water-soluble protein. Thus, for example, the two or three components mentioned above are made to occur in an ampule or vial by dissolving or suspending them in sterile water or sterile physiological saline. In this case, the method of preparation may comprise admixing a solution of the pharmacologically active oligopeptide salt and further, if necessary, a solution of the additive or adding the additive in a powder form to a solution of the pharmacologically active oligopeptide salt or any other combination of adequate procedures. The dosage form may also be prepared by adding sterile water or sterile physiological saline to a lyophilizate or vacuum-dried powder in which the pharmacologically active oligopeptide salt, and if necessary the additive, coexist. This unit dosage form may contain one or more conventional additives such as pH adjusting agents (e.g. glycine, hydrochloric acid, sodium hydroxide), local anesthetics (e.g. xylocaine hydrochloride, chlorobutanol), isotonizing agents (e.g. sodium chloride, mannitol, sorbitol), emulsifiers, adsorption inhibitors (e.g. Tween® 60 or 80), talcum, starch, lactose and tragacanth, magnesium stearate, glycerol, propylen glycol, preserving agents, benzyl alcohol, methylhydroxy benzoate and/or oleum arachid hydrogen. This unit dosage form may further contain pharmaceutically acceptable excipients such as polyethylene glycol 400 or dextran.

The composition of the present invention is made by admixing these ingredients according to a conventional method. The goal of admixing the ingredients of the present composition should be such that the activity of the pharmacologically active oligopeptide is maintained and bubble formation minimised during the process. The ingredients are put into a vessel (for example a bottle or drum) either at the same time or in any order. The atmosphere in the vessel can be, for example, sterile clean air or sterile clean nitrogen gas. The resultant solution can be transferred to small vials or ampules and can be further subjected to lyophilization.

The liquid form or the lyophilizate powder form of the composition of the present invention may be dissolved or dispersed in a solution of a biodegradable polymer such as poly(lactic-glycolic) acid copolymer, poly(hydroxybutyric acid), poly(hydroxybutyric-glycolic) acid copolymer, or the mixture of these, and then may be formulated, for example, to films, microcapsules (microspheres), or nanocapsules (nanospheres), particularly in the form of soft or hard capsules.

In addition, the composition of the present invention encapsulated in liposomes comprising phospholipids, cholesterol or the derivatives of these can be further dispersed in physiological saline or a hyaluronic acid solution dissolved in physiological saline.

The soft capsule may be filled with the liquid form of the composition of the present invention. The hard capsule may be filled with the lyophilizate powder of the composition of the present invention, or the lyophilizate powder of the present composition may be compressed to tablets for rectal administration or oral administration respectively.

Of course, the composition of the present invention can be supplied in a pre-filled syringe for self-administration.

The present invention also relates to the use of the oligopeptides of the present invention and the wild type TPO-Rp, the nucleotide sequence encoding these oligopeptides, the vector of the present invention, the host cell of the present invention and/or the antibody of the present invention for the preparation of a medicament for diagnosing or treating hematological disorders, in particular thrombocytopenia.

Finally, the present invention relates to diagnostic compositions for diagnosing hematological disorders, in particular thrombocytopenia comprising the oligopeptide of the present invention or wild type TPO-Rp, the nucleotide sequence encoding these oligopeptides, the vectors of the present invention, the host cell of the present invention and/or the antibodies of the present invention, optionally in conjunction with an acceptable carrier. In a particularly preferred embodiment of the present invention, the above mentioned agents used in the diagnostic composition of the present invention may be labelled according to the above. Thus, labelled oligopeptides, labelled nucleotide sequences, labelled cells and/or labelled antibodies of the present invention may be used to specifically detect TPO-R related conditions, in particular disorders. Similarly, and as explained above, the labelled agents may be used to identify and isolate potential further drugs.

Although only preferred embodiments of the invention are specifically described above, it will be appreciated that modifications and variations of the invention are possible without departing from the spirit and intended scope of the invention. Further preferred embodiments of the present invention are listed in the claims.

SEQ ID Nos. 1 to 7 represent the amino acid sequences of the oligopeptides of the present invention.

The peptide length is identified by the first and last amino acid number according to the position in full length (also called wild type) TPO-Rp, as disclosed in WO 99/42127, which is with respect to the TPO-Rp wild type amino acid sequence and its preparation fully incorporated in the present teaching. Single amino acid substitutions were named by the conventional nomenclature, e.g. "R9A" - describing that the original Arginine residue at position 9 of TPO-Rp wild type has been replaced with Alanine.

The figures show:

Fig. 1        the effect of the present oligopeptides in carboplatin (180 mg/kg) treated mice,

Fig. 2        the effect of the present oligopeptides in carboplatin (200 mg/kg) treated mice,

Fig. 3 to 6       the results of stability studies in form of HPLC profiles,

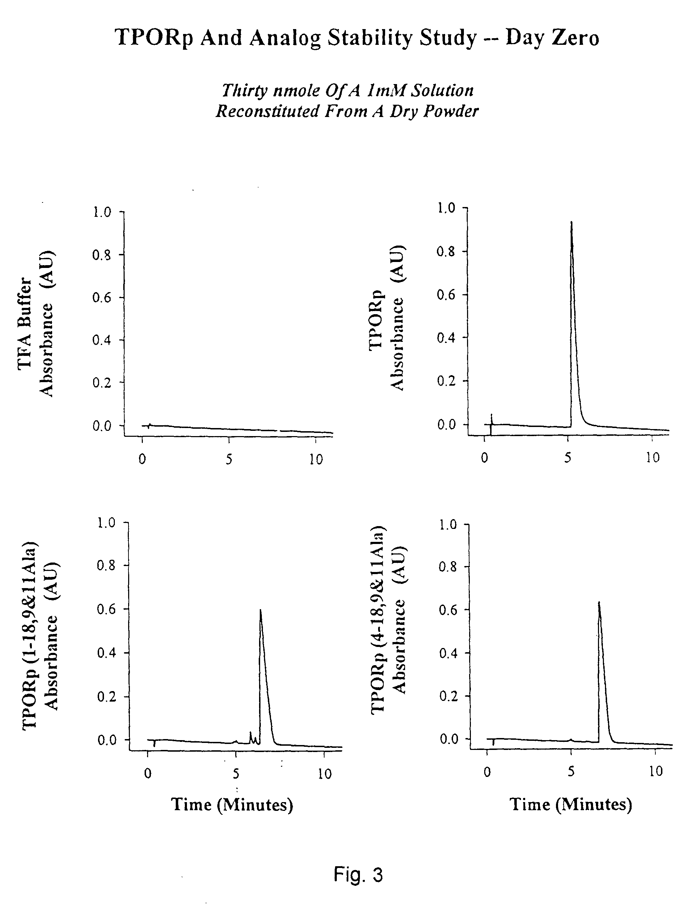

(Fig. 3:       day 0, 30 nmole of a 1 mM solution reconstituted from dry powder,

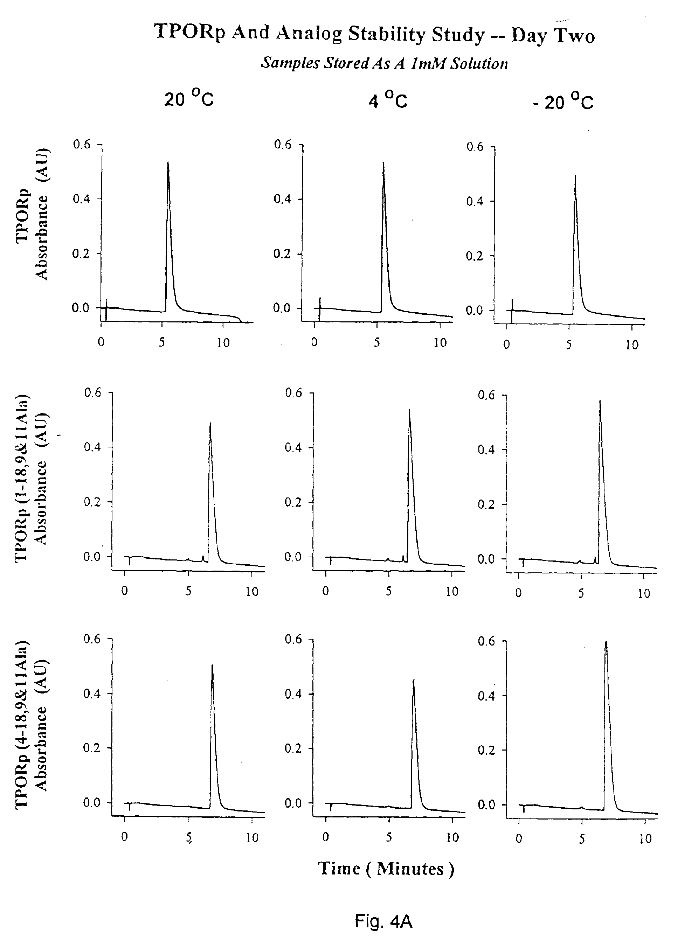

Fig. 4A:       day 2, samples stored as a 1 mM solution,

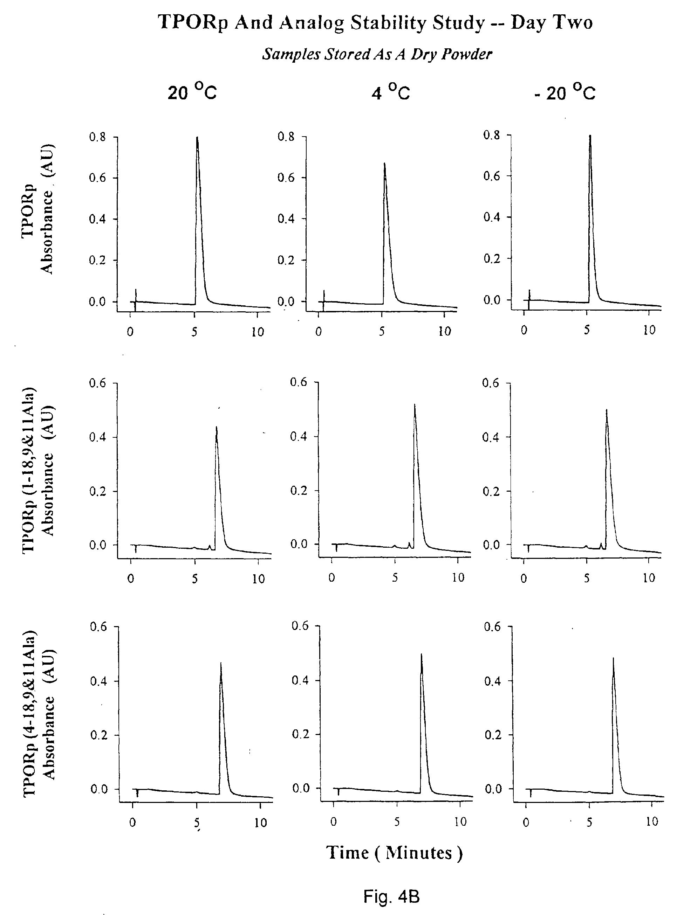

Fig. 4B:       day 2, samples stored as a dry powder,

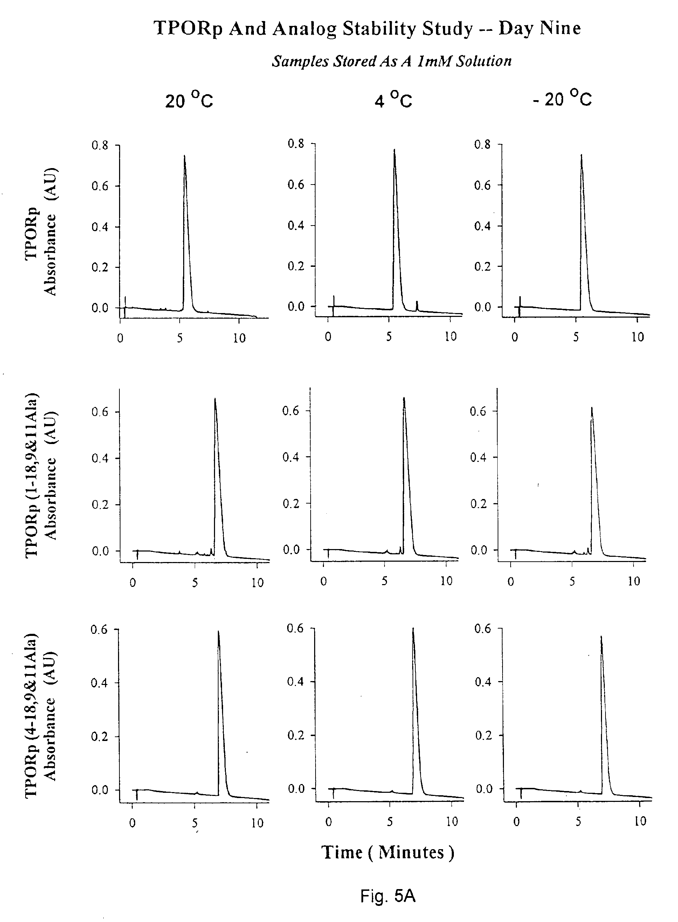

Fig. 5A:       day 9, samples stored as a 1 mM solution,

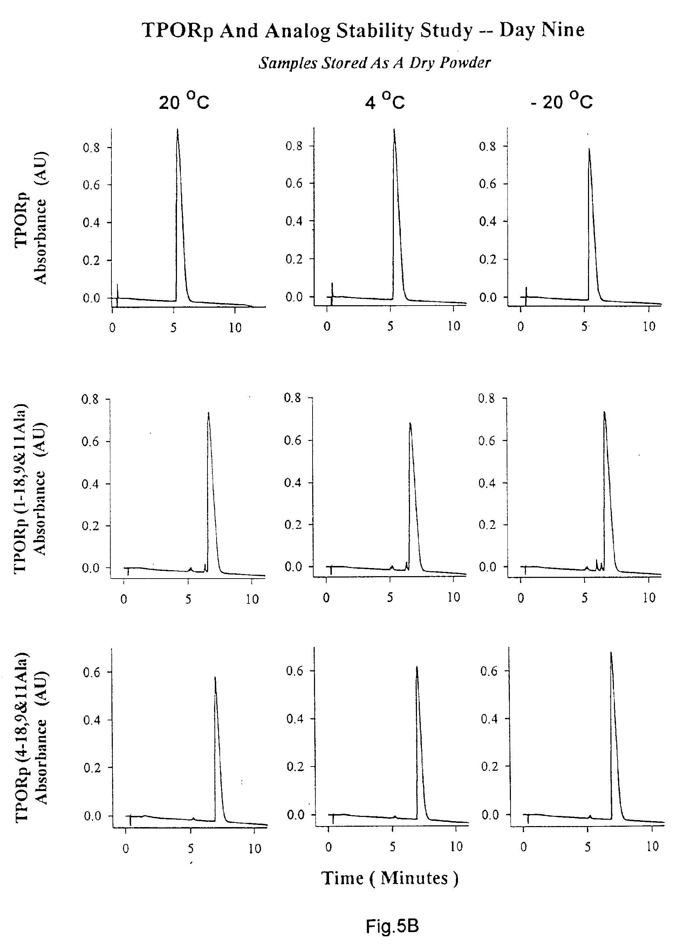

Fig. 5B:       day 9, samples stored as a dry powder,

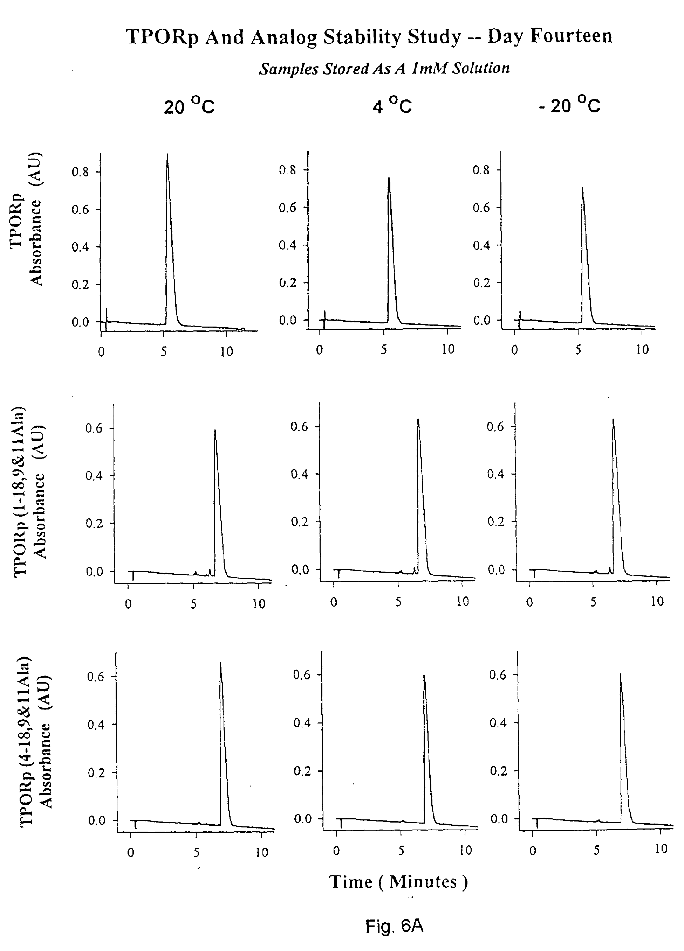

Fig. 6A:     day 14, samples stored as a 1 mM solution,

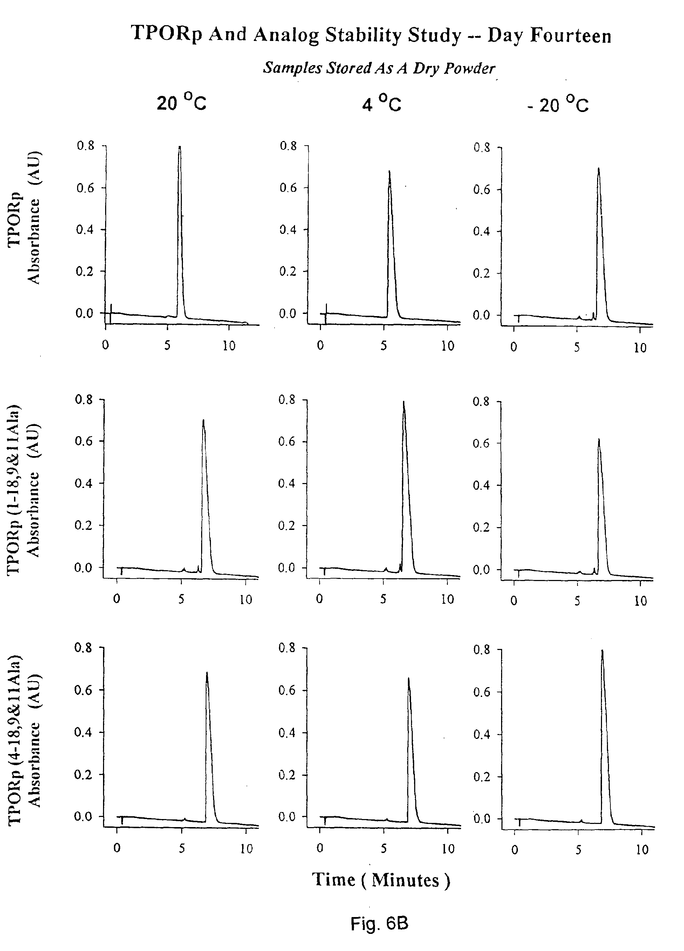

Fig. 6B:     day 14, samples stored as a dry powder.)

Example 1:

Effect of TPO-Rp (wild type and present oligopeptides) in treating carboplatin induced thrombocytopenia: I

Methods:

The model used for the in vivo experiments was reported previously (Akahori et al. (1996) Stem Cells 14: 678-689, Akahori et al. (1996) Br. J. Haematol. 94:722-728, Shibuya et al. (1998) Blood 91:37-45, Andrews et al. (1996) Stem Cells 14:661-677). Improvements and modifications that were introduced are indicated below.

Dosing schedule

Each test group (PBS, TPO, or TPO-Rp) was given as a single daily dose administered by intraperitoneal injection. Daily doses were begun on day 0 and continued throughout the duration of the experimental period (day 10). Two i.p. injections of carboplatin 90 mg/kg each (PBS for group 11) were given on days 0 and 4.

Blood sampling

Blood collections were taken on days -0 (basal), 8, 11, 14, and 18. Blood was obtained by sampling from the tail of mice (8 week old male C57BL/6Y mice), followed by cauterisation. A volume of 80 μl of blood from each mouse was collected into a heparinized blood collection tube. The 80 μl of blood was then mixed with 160 μl of saline containing sufficient EDTA (0.225%) to provide for anticoagulation.

The effect of the TPO-Rp with SEQ ID No. 2 (TPO-Rp, 1-18, R9A, R11A) and No. 4 (TPO-Rp, 4-18, R9A, R11A) in comparison to the wild-type peptide (TPO-Rp wild type, amino acid sequence: A R G G T L E L R P R S R Y R L Q L R A R L N) in vivo was tested in carboplatin induced thrombocytopenia in mice. Carboplatin is used to induce thrombocytopenia. In order to work with a model that is as similar as possible to clinical situations, a previously reported carboplatin model (Akahori et al. (1996) Stem Cells 14:678-689, Akahori et al. (1996) Br. J. Haematol. 94:722-728, Shibuya et al. (1998) Blood 91:37-45, Andrews et al. (1996) Stem Cells 14:661-677) has been carefully studied and modified accordingly. Additionally, a method for animal bleeds has been carefully studied, in order to work with animals that show the highest response to a given drug and which are at the same time under a minimum of stress (see Methods above). Thus, the dose of carboplatin has been increased to 180 mg/kg and was given by two intraperitoneal (i.p) injections on day 0 (half-dose) and day 4 (second half-dose). Carboplatin induced severe thrombocytopenia on day eight.

EP 1 149 906 A1

The animal groups used are presented in table I.

Table I.

| Experimental groups in "in vivo" studies. | | |
|---|---|---|
| Group | Total dose | Animals |
| 1 | Carboplatin[a]+TPO-Rp wild type 0.03 mg/kg/day | 8 |
| 2 | Carboplatin[a]+TPO-Rp wild type 0.30 mg/kg/day | 8 |
| 3 | Carboplatin[a]+TPO-Rp wild type 0.90 mg/kg/day | 8 |
| 4 | Carboplatin[a]+TPO-Rp SEQ ID No. 2 0.03 mg/kg/day | 8 |
| 5 | Carboplatin[a]+TPO-Rp SEQ ID No. 2 0.30 mg/kg/day | 8 |
| 6 | Carboplatin[a]+TPO-Rp SEQ ID No. 2 0.90 mg/kg/day | 8 |
| 7 | Carboplatin[a]+TPO-Rp SEQ ID No. 4 0.03 mg/kg/day | 8 |
| 8 | Carboplatin[a]+TPO-Rp SEQ ID No. 4 0.30 mg/kg/day | 8 |
| 9 | Carboplatin[a]+TPO-Rp SEQ ID No. 4 0.90 mg/kg/day | 8 |
| 10 | Carboplatin[a]+TPO 2.4 µg/kg/day | 8 |
| 11 | No carboplatin | 8 |
| 12 | Carboplatin[a] alone | 12 |

[a]90 mg/kg of carboplatin given on days 0 and 4.

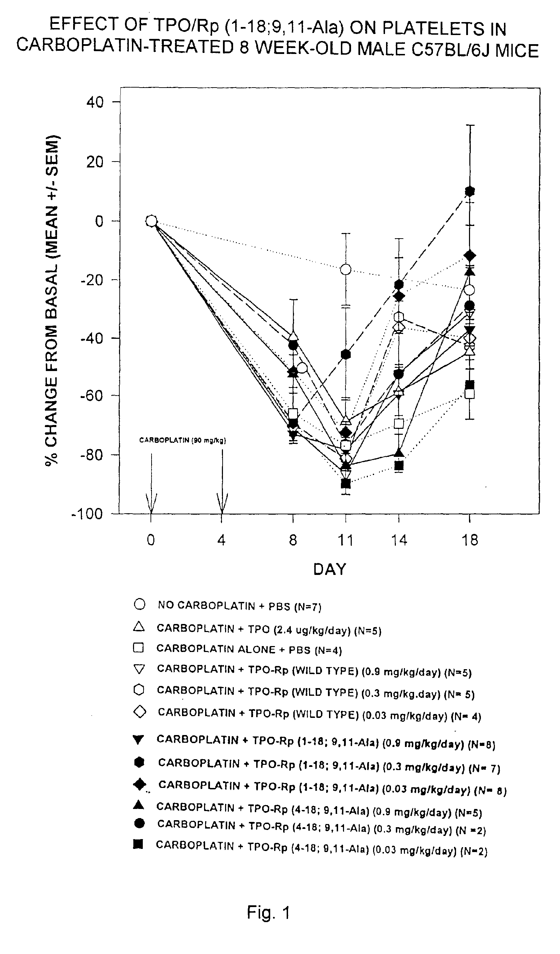

Figure 1 shows a severe drop in platelet number in carboplatin treated mice, and summarises the experimental results for the above mentioned different experimental groups. The results are expressed in percent change from basal values of individual animals. Figure 1 shows that the wild type peptide TPO-Rp in a dose of 300 µg/kg/day and 30 µg/kg/day significantly increased the thrombocyte number. It should be mentioned that at this very high carboplatin dose, TPO at the concentration used (2.4 (µg/kg/day), which had previously shown an effect, was unable to prevent a decrease in platelets. Figure 1 shows also that the shortened TPO-Rp peptide with SEQ ID No. 2 i.e. residues of 1-18 and with R9A, R11A had a strongly protected carboplatin-induced platelets drop at two doses: 300 µg/kg/day and 30 µg/kg/day. The protective effect caused by two doses is equally remarkable; however based on the survival rate (see below), the lowest dose of 30 µg/kg/day has a particular significance, while the dose of 300 µg/kg/day has comparable effects to the TPO-Rp wild type. The highest peptide dose of 0.9 mg/kg/day showed a very low effect on platelet protection, which could possibly be attributed to peptide aggregation. Figure 1 also shows that the shortest version of the TPO-Rp peptide with SEQ ID No. 4, i.e. residues with 4-18, with R9A, R11A, showed some effect on platelet levels in carboplatin treated animals. None of the peptides used, wild type TPO-Rp, TPO-Rp 1-18 (R9A, R11A) and TPO-Rp 4-18 (R9A, R11A), have significant effects on other blood components. Further, none of the treatments affect weight in the animals.

It is important to examine the survival rate of treated animals. Statistically evaluated by CHI-square analysis, the most significant effect and thus the highest survival rate is observed with the TPO-Rp, 1-18 (R9A, R11A) peptide. Treatment with the wild type peptide, as well, has a statistically significant survival rate.

All of the above clearly indicates that the peptide TPO-Rp 1-18 (R9A, R11A) significantly restores carboplatin-induced thrombocytopenia and represents an improvement of 10 times in "in vivo" activity of the compound.

Example 2:

Effect of TPO-Rp (wild type and present oligopeptides) in treating carboplatin induced thrombocytopenia: II

Methods: Dosing Schedule

Each test group was given a single daily dose administered by intraperitoneal injection. Daily doses were begun on day 0 and continued throughout the duration of the experimental period (day 13). Minimal doses were given on indicated days: 0 and 4; 0, 4 and 8; 0, 4, 8 and 12; days 8 through 14. Two i.p. injections of carboplatin 100 mg/kg were given on days 0 and 4.

Blood sampling

Blood collections were taken on days 0 (basal), 8, 11, 14 and 18. Blood was obtained by sampling from the tails of mice (8 week old male C57BL/6J mice) followed by cauterisation. A volume of 80 µl of blood from each mouse was collected into a heparinized blood collection tube. The 80 µl of blood was then mixed with 160 µl of saline containing sufficient EDTA (0.225%) to provide for anticoagulation.

The dose of carboplatin has been increased to 200 mg/kg and was given as a cumulative dose by two intraperitoneal (i.p.) injections on day 0 (half-dose) and day 4 (second half-dose). Carboplatin induced severe thrombocytopenia on day eleven.

Animal groups used are presented in Table II.

Table II

| Experimental groups in "in vivo" studies | | |
|---|---|---|
| Group | Total dose | Dosing schedule (days) |
| 1 | Carboplatin[a]+TPO-Rp SEQ ID No. 2, 0.3 mg/kg/day | 0 through 13 |
| 2 | Carboplatin[a]+TPO-Rp SEQ ID No. 2, 0.3 mg/kg/day | 0 |
| 3 | Carboplatin[a]+TPO-Rp SEQ ID No. 2, 0.3 mg/kg/day | 0 and 4 |
| 4 | Carboplatin[a]+TPO-Rp SEQ ID No. 2, 0.3 mg/kg/day | 0, 4 and 8 |
| 5 | Carboplatin[a] +TPO-Rp SEQ ID No. 2, 0.3 mg/kg/day | 0, 4, 8 and 12 |
| 6 | Carboplatin[a] +TPO SEQ ID No. 2, 0.3 mg/kg/day | 8 and 14 |
| 7 | Carboplatin[a] +TPO, 2.4 µg/kg/day | 0 through 13 |
| 8 | Carboplatin[a]+TPO, 10 µg/kg/day | 0 through 13 |
| 9 | Carboplatin[a] +TPO, 10 µg/kg/day | 8 through 14 |
| 10 | No carboplatin | - |
| 11 | Carboplatin[a] alone | 0 and 4 |

[a] 100 mg/kg of carboplatin given on days 0 and 4

Results:

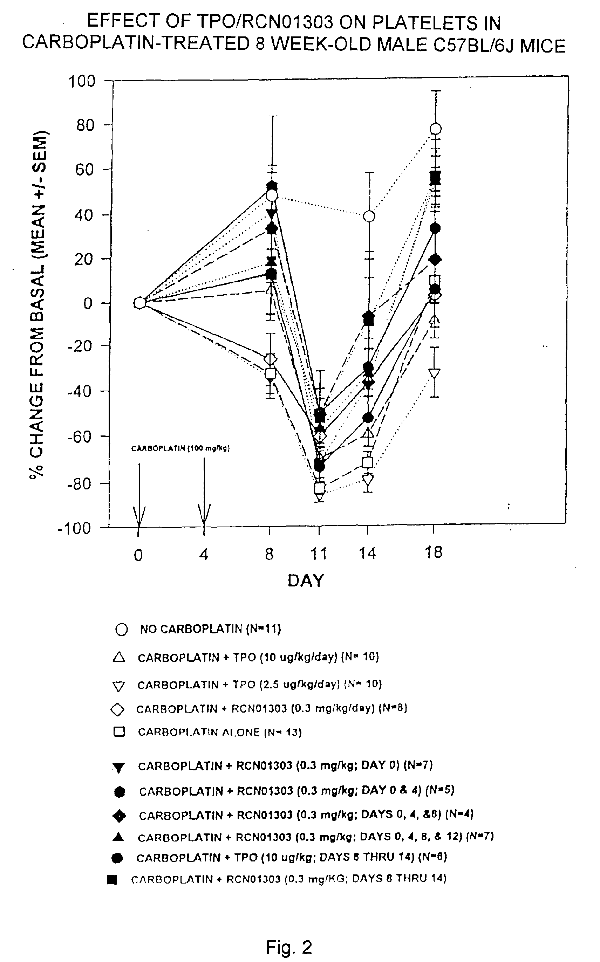

Figure 2 summarises the results of carboplatin treated mice for all experimental groups (RCN-O1303 represents the oligopeptide with SEQ ID No. 2). The results are expressed in percent change from basal platelets values of individual animals.

Figure 2 shows that the peptide in a dose of 300 µg/kg/day increases the thrombocyte number. It should be mentioned that the carboplatin dose has been increased compared to Example 1 in order to test the compound potency in the case of severe thrombocytopenia, which is a very common clinical situation. TPO hormone used at concentrations of 2.4 µg/kg/day - as in Example 1 with a dose of carboplatin 180 mg/kg - was again unable to protect decrease in platelets. At the higher dose of 10 µg/kg/day, TPO showed some effect that does have statistical significance. However, the protective effect caused by the oligopeptide with SEQ ID No. 2 is greater than that caused by TPO in this particular carboplatin model.

Figure 2 also shows a summary of the results where the possibility of reduced oligopeptide dosing was tested. The oligopeptide at a concentration of 0.3 mg/kg/day was given on day 0 only; days 0 and 4; 0, 4 and 8; and 0, 4, 8, and 12. Remarkably, it seems that peptide dosing on days 0 and 4 only was sufficient to show protection in carboplatin treated mice. Statistically significant levels of protection were observed on days 11 and 14. It is of interest to note that those groups of minimal treatment show great variability in response. Such a situation is not uncommon, as it is known that individuals respond differently to the same treatment. Nevertheless, there is a statistically significant effect caused by the oligopeptide with SEQ ID No. 2 when given on days 0 and 4 only. As well, dosing on days 0, 4 and 8 or 0, 4, 8 and 12 showed significant effects on thrombocytopenia. The effect observed with this minimal dosing is comparable to the effect with continuous administration (days 0 through 13) of the oligopeptide. Such a possibility of reduced dosage, but the same therapeutic effect, could clearly be a clinical advantage.

Studies with wild type TPO-Rp and the present oligopeptides have indicated that the peptide mechanism of action is different from that of the natural hormone. There appears to be a lack of TPO effect on patients that first have received the chemotherapy and, several days later, have been treated with TPO. Accordingly, the following experiment was carried out.

A group of carboplatin treated animals that were given the chemotherapeutic agent from day 0 received TPO at a dose of 10 µg/kg/day from day 8 through 14. Similarly, in another group, the oligopeptide with SEQ ID No. 2 at 0.3 mg/kg/day was given from day 8 through day 14 to carboplatin treated animals that started treatment on day 0. Results are also shown in Figure 2. TPO did not show a significant effect on thrombocytopenia. In contrast, TPO-Rp (SEQ ID No. 2) has shown the rescue in platelet level, despite the fact that it was given after the start of the therapy. A statistically significant increase in the platelet level in oligopeptide treated animals suggests that its protective effect is not only present if given at the beginning of the therapy. This finding not only shows the ability of the oligopeptide of the present invention to prevent the damaging effect of carboplatin when given after the start of the therapy, but also indicates that the oligopeptide has a different mechanism of action than TPO. Thus, both effects - (i) minimal dosing schedule and (ii) different mechanisms of action - provide considerable advantages.

Example 3:

Pharmacokinetics of oligopeptides ex vivo

Methods:

The degradation profiles of wild type TPO-Rp, TPO-Rp 1-18 (R9A, R11A, SEQ ID No. 2) and TPO-Rp 4-18 (R9A, R11A, SEQ ID No. 4) were studied in human serum (a pool of serum from 200 patients) and rat plasma (a pool of plasma from 50 animals). In order to enable detection of degradation $^{125}$I-labelled peptides (labelled at Y14) have been used. Only the intact peptide and labelled degradation products are detected by HPLC analysis. Therefore, such analysis performed at different points clearly detects when a given peptide is degraded. Incubation was performed at 37°C. The concentration of labelled peptide was $\sim$ 1 µM in these studies.

Results:

The results of the peptide degradation in pooled rat plasma are presented in Table III. The half-life of TPO-Rp is seen to be $\sim$ 12 minutes. TPO-Rp 1-18 with SEQ ID No. 2 showed a slightly shorter half-life of $\sim$ 1.1 minutes, whereas the oligopeptide with SEQ ID No. 4 showed a very short half-life, $\sim$ 0.5 minutes, in rat plasma.

Table III:

| Ex vivo stability of peptides at 37°C. **Peptides half-life (minutes)** | | |
|---|---|---|
| **Peptide** | **Pooled rat plasma** | **Pooled human serum** |
| TPO-Rp, wild type | 12.1 | 1.9 |
| TPO-Rp (1-18; R9A,R11A) | 1.1 | 9.6 |
| TPO-Rp (4-18; R9A, R11A) | 0.5 | 4.9 |

The results of the peptide degradation in pooled human serum are also shown in Table III. As opposed to rat plasma, in human serum the TPO-Rp wild type peptide has a half-life of $\sim$ 1.9 minutes. TPO-Rp 4-18 (R9A, R11A) has improved stability and a half-life of $\sim$ 4.9 minutes. Considerably longer half-life was obtained with the oligopeptide 1-18 (R9A, R11A) - a half-life of $\sim$ 9.6 minutes, which is a five-fold improved stability over the wild type oligopeptide. This represents a significant improvement, as most peptides have a relatively short half-life of 2-3 minutes. It should be noted that TPO-Rp 1-18 R9A, R11A shows in vivo activity improvements over wild type TPO-Rp despite its shorter half-life in rat plasma.

In vivo degradation studies of TPO-Rp 1-18 R9A, R11A (3 mg/kg) in rats after intravenous bolus administration showed a calculated half-life in blood of 7.3 minutes.

Example 4:

Peptide stability

Methods:

The HPLC method used was based on UV detection of the sample during elution from a reverse phase column system, C8 MICROSORB MV column (Rainin Instrument Company). The two buffer system begins with 9.5% 5 mM TFA (buffer A) and 5% ACN (buffer B). After sample injection, the percent of B is rapidly increased to 10% and then gradually increased to 35% over 10 minutes. The sample elution period is followed by a brief column wash with an increase of buffer B to 90%.

Results:

The stability of wild type TPO-Rp, TPO-Rp 1-18 (R9A, R11A, SEQ ID No. 2) and TPO 4-18 (R9A, R11A, SEQ ID No. 4) was addressed by HPLC analysis after storage of the three peptides at the following three temperatures: room temperature ($\sim$ 20°C), 4° C and -20° C. The peptides were stored as either 1 mg of dry powder or as a 1 mM solution in water, with no excipients in either condition. After establishing the HPLC elution profiles of these three peptides on day 0 the peptide, HPLC elution profiles were re-tested on days two, nine and fourteen.

Figure 3 shows HPLC profiles for 30 nmole of wild type TPO-Rp and the shorter oligopeptides at the beginning of the study when 1 mM peptide solutions were prepared from a dry powder. Figure 4 shows HPLC profiles of peptides stored as 1 mM solution (Fig. 4A, or dry powder Fig 4B) on day 2 of the study. Day 9 of the peptide stability study, as shown on Figure 5A and 5B, shows no changes in peptide HPLC profiles, thus indicating great peptide stability when stored at room temperature, 4°C or $\sim$ 20°C. Remarkably, on day 14 of the peptide stability study, Figure 6A and 6B, wild type TPO-Rp and the two oligopeptides of the present invention showed unchanged HPLC profiles. No signs of peptide degradation were detected when stored as a 1 mM solution, or as a powder, at room temperature, 4° C or $\sim$ 20°C. It could be concluded that TPO-Rp and the oligopeptide of the present invention could be stored as a 1 mM solution in water or dry powder, at room temperature, 4° C or $\sim$ 20° C, for a period of two weeks without any sign of a peptide degradation.

Further studies showed that wild type oligopeptide TPO-Rp and the peptide with SEQ ID No. 2 prepared as a 1 mM solutions in water, saline or saline with 0.1% human albumin kept at -20°C, 4°C and room temperature for 1, 3 and 4 weeks showed no sign of degradation in HPLC studies.

Example 5

Formulation of TPO-Rp 1-18 R9A, R11A and manufacturing procedure

## 5A) Vehicle solution

| Ingredients | % w/v | mg/ml | 4L batch |
|---|---|---|---|
| Sodium Acetate, Trihydrate, USP 10 mM (F.W. 136.08) | 0.136 | 1.361 | 5.444 gm |
| Water for Injection, USP q.s. | 100 | 1 mL | 4 Liters |
| pH* | 5.3 ± 0.2 | | |

*(10N HCl and then 1N HCL or 1N NaOH solution for pH adjustment if needed)

| | | | |
|---|---|---|---|
| Mannitol, USP | 5.0 | 50 | 200 gm |

Manufacturing Procedure:

1. 90% (3.6 liters) of water for injection (WFI) are added into a suitable glass container.

2. 5.444 gm Sodium Acetate, Trihydrate are added into #1.

3. pH of #2 was adjusted with 10N HCl (approx. 600 ul) and additional 1N HCl or 1N NaOH solution if needed to pH 5.3 ± 0.2.

4. 200 gm Mannitol was added and dissolved into #3. It was mixed with gentle stirring until it was completely dissolved.

5. A sufficient quantity of WFI was added into #4 to final volume of 4 liters. It was mixed with stirring until it was homogeneous.

6. Under aseptic condition, solution #5 was filtered through 0.2 micron membrane into a sterile container.

7. The solution (100 mL/vial) was asceptically filled into seven type I glass vials prepared according to sterilization procedure. In addition two similarly sterilized vials (10 mL per vial) were filled as initial and retain samples. It was sealed with Teflon gray butyl stopper and aluminum seal.

| 5B) Injectable Solution (1 mg/mL) | | | |
|---|---|---|---|
| Ingredients | % w/v | mg/ml | 600 mL batch |
| TPO-Rp 1-18, R9A, R11A (anhydrous free base) powder* | 0.1 | 1 | 0.66 gm* |
| Vehicle see 5A | 100 | 1 mL | 600 mL |

Note: (for 5B, 5C, 5D)
TPO-Rp 1-18, R9A, R11A is supplied as hydrous acetate salt. The amount is calculated as follows:

$$\text{Amount of peptide powder (hydrous acetate salt)} = \frac{\text{Amount of free base required}}{\text{\% Peptide content}}$$

*Based upon peptide 90.7% content

Manufacturing procedure

1. An exact amount of the vehicle solution (600 mL) was added to a suitable glass container.

2. An exact amount of oligopeptide powder (0.66 gm powder) was added and dissolved into #1 and mixed well.

3. The pH of solution #2 was measured and adjusted to pH $5.3 \pm 0.2$, if needed.

4. Solution #3 was sterile filtered through 0.2 micro membrane (Milipore Durapore membrane or equivalent), under aseptic conditions.

5. The solution (75 mL/vial) was aseptically filled into seven sterile type I glass vials prepared according to sterilization procedure. An additional two similarly sterilized vials (10 mL per vial) were also filled as initial and retain samples. Sealing was done with Teflon faced gray butyl stopper and aluminum seal.

| 5C) Injectable Solution (5 mg/mL) | | | |
|---|---|---|---|
| Ingredients | % w/v | mg/ml | 600 mL batch |
| TPO-Rp 1-18, R9A, R11A (anhydrous free base) powder* | 0.5 | 5 | 3.3 gm* |
| Vehicle see 5A | 100 | 1 mL | 600 mL |
| Manufacturing procedure: see 5B with modification in quantities according to above table. | | | |

| 5D) Injectable Solution (9 mg/mL) | | | |
|---|---|---|---|
| Ingredients | % w/v | mg/ml | 650 mL batch |
| TPO-Rp 1-18, R9A, R11A (anhydrous free base) powder* | 0.9 | 9 | 6.45 gm* |
| Vehicle see 5A | 100 | 1 mL | 650 mL |
| Manufacturing procedure: see 5B with modification in quantities according to above table. | | | |

Example 6:

Methods: "In vitro" signalling study

Treatment of the cells: TF-1 cells were grown to an approximate density of $1 \times 10^6$ cells in RPMI 1640 media with 1x Penicillin/Streptomycin, 2mM glutamin, 10% fetal bovine serum (Hyclone) and 1 ng/ml GM-CSF, centrifuged down and resuspended in media with 3% serum and no GM-CSF. The cells were starved for 14-18 hours at 37°C (5% $CO_2$), spun down and resuspended at a density of $1 \times 10^6$ cells/ml in a medium without serum and GM-CSF. 2 ml of cell suspension was treated with the oligopeptide or TPO (as indicated per experiment) at 37°C for 30 min. (5% $CO_2$). 10 ml of ice-cold cell wash buffer was added per Falcon tube and centrifuged down quickly at 4°C and 3000 rpm,. The medium was carefully aspirated and the wash repeated twice with PBS.

The following steps were performed on ice. The medium was aspirated and 0.6 ml of 2 x lysis buffer per tube was added. This was pipetted up and down and transferred to Eppendorf tubes, placed on ice for circa 30 min., and centrifuged at 14,000 x g for 10 min. The supernatant (lysate) was used in immunoprecipitations.

Immunoprecipitations: 20-40 μL of GammaBind G Sepharose slurry was used per immunoprecipitation. The protein G beads were washed several times with 0.5 x lysis buffer in Eppendorf tubes and 1-4 μg of PY-99 antibody (Santa Cruz-Biotechnology) was added per immunoprecipitation. The tubes were incubated with end-over-end rotation for 2 h at room temperature. The lysate was added and the tubes further incubated with end-over-end rotation overnight at 4°C. The beads were washed 3 times with 1x lysis buffer and once with 0.5 M Tris pH 6.5. 50 μl of SDS-sample buffer was added and the samples were boiled for 3 min prior to being applied to the gels.

Western blot analysis: about 10 μl/sample was run on a 8% Polyacrylamide mini gel, transferred to PVDF membrane (Millipore), blocked for 1 h in blocking buffer and incubated with αSTAT5 antibody (Santa Cruz-Biotechnology) overnight at 4°C in an 1:1000 dilution. The membrane was washed, incubated with appropriate alkaline phosphatase conjugated secondary antibody, 1:2000 dilution at room temperature for 2 h. The PVDF-membrane was washed with Blotto and developed with NBT/BCIP (Cappel).

Results:

It was evaluated whether the oligopeptides that did not show any apparent degradation by HPLC analysis (see Example 4) still maintain their biological activity.

The activity of the oligopeptides of the present invention and wild type TPO-Rp was examined by in vitro signalling assays. Using the above identified standard procedures, TF-1 cells were stimulated with peptide samples that underwent HPLC analysis. Phosphorylation and thus activation of STAT5, a substrate of JAK2 kinase that is activated by TPO-R signal transduction, was measured. Peptides were tested at 3 μ M concentrations on "day 18" of the stability study and at 50 nM and 5 μM on "day 30" of the stability study. All the peptides were evaluated three to four times in independent experiments. All the data were collected, Western blots were scanned, and intensity of the STAT5 phosphorylation was quantified.

Data from all experiments (mean +/- SEM) is presented in Table IV to IX (see below) (W: water, S: saline, H: 0,1% HSA) The results indicate some differences in the peptide's activity when stored under different conditions. On "day 18" of the stability study, the TPO-Rp (1-18, R9A, R11A) peptide showed significantly diminished activity when stored at room temperature. The activity was maintained when the compound was stored at 4°C or -20°C. Although the activity was preserved when the peptide solution was prepared in water or saline, the compounds activity was remarkably best preserved with addition of 0.1% HSA. This observation was confirmed with the results from the "day 30" of the stability study. The peptide with SEQ ID No. 2 remains active after 30 days when dissolved in water and stored at -20°C. When stored in saline or saline plus 0.1% HSA, the solution remains active when stored at both temperatures, 4°C and -20°C. By way of comparison, on "day 30" of the stability study, the wild type TPO-Rp showed a significant decrease in activity when stored as a solution in water at -20°C.

The above stability studies revealed that TPO-Rp (1-18, R9A, R11A, SEQ ID No. 2) is a rather stable molecule which, when stored at -20°C in any solvent, maintains activity for a month. Interestingly, although no degradation pattern of the peptide was observed under any solvent or temperature condition, the activity assay has revealed some differences.

Example 7:

Biological activity of shortened TPO-Rp oligopeptides

Methods:

The oligopeptides with SEQ ID Nos. 1 to 7 were synthesised by solid phase synthesis and subsequent preparative HPLC purification to 90-95% purity.

The identity of the oligopeptide is tested by mass-spectrometry and amino-acid analysis. The indicated MW is from mass spectroscopy (M+H[+]) (SEQ ID No. 1: 2141, No. 2: 1971, No. 3: 1857, No. 4: 1687, No. 5: 2240, No. 6: 2069, No. 7: 2736, wild type: 2755).

Dose-response curves of the present oligopeptides were performed and their activity was compared to the wild type TPO-Rp.

Peptide dose response activity was evaluated primarily by in vitro signalling assays. Phosphorylation and

**EP 1 149 906 A1**

thus activation of STAT5, a substrate of JAK2 kinase that is activated by TPO-Rp signal transduction, was measured. Each oligopeptide activity was addressed through a broad range of concentrations. Peptides were tested at concentrations of 0.3, 3, 10, 30 nM and 0.1, 0.3, 3 and 30 μM TPO-Rp. Oligopeptide activity as a measurement of STAT5 phosphorylation in each experiment was compared to activity obtained with 10 ng/ml of TPO, a hormone concentration that gives a maximal activation and signalling through TPO-R. All the peptides were evaluated four to five times in independent experiments. All the data was collected, Western blots were scanned and the intensity of the STAT5 phosphorylation was quantified.

Results:

The activity of each oligopeptide was addressed as percent of its maximum activity; thus 100% activity was assigned to the value of STAT5 protein phosphorylation obtained with 30 μM oligopeptide concentration.

The results clearly indicate that all of the oligopeptides have retained activity comparable to the wild type TPO-Rp. Table X (see below) summarises approximate $EC_{50}$ values for all tested oligopeptides.

The first set of oligopeptides (A1 - L18, SEQ ID Nos. 1 and 2) showed not only activity comparable to the wild type TPO-Rp, but also improvement in potency with its R9A, R11A form (approx. $EC_{50}$ 10 nM). Remarkably, this part of the oligopeptide was shown to be crucial for TPO-Rp activity when addressed through Alanine walk and structural analysis. The activity increase may be due to improvement of oligopeptide structure, stability or both.

The shortest oligopeptide, 15 amino acids long (G4-L18, SEQ ID No 3 and 4) retained activity comparable to wild type TPO-Rp in both forms. The third set of oligopeptides (G4-R21, SEQ ID Nos. 5 and 6) showed activity identical to wild type TPO-Rp in both forms.

Table IV

| Activity of peptides used in stability studies TPO-Rp (1-18, R9A, R11A) at 3 μM; "day 18" stability study | | | | | | |
|---|---|---|---|---|---|---|
| Condition | | STAT5 activation measured by protein phosphorylation (arbitrary units) | | | | |
| Temperature (°C) | Solvent | Exp #1 | Exp #2 | Exp #3 | Mean | SD |
| **-20** | W | 5.6 | 7.8 | 6.2 | **6.5** | 1.1 |
| **4** | W | 5.9 | 6.5 | 7.8 | **6.7** | 1.0 |
| **25** | W | 3.4 | 2.8 | 2.5 | **2.9** | 0.5 |
| **-20** | S | 6.5 | 8.9 | 7.6 | **7.7** | 1.2 |
| **4** | S | 5.4 | 6.2 | 4.9 | **5.5** | 0.7 |
| **25** | S | 2.4 | 1.9 | 2.2 | **2.2** | 0.3 |
| **-20** | H | 10.7 | 11.0 | 12.4 | **11.4** | 0.9 |
| **4** | H | 10.3 | 11.5 | 12.1 | **11.3** | 0.9 |
| **25** | H | 2.2 | 1.8 | 1.5 | **1.8** | 0.4 |

Table V

| TPO-Rp (wild type) at 3 μM; "day 18" stability study | | | | | | |
|---|---|---|---|---|---|---|
| Condition | | STAT5 activation measured by protein phosphorylation (arbitrary units) | | | | |
| Temperature (°C) | Solvent | Exp #1 | Exp #2 | Exp #3 | Mean | SD |
| **-20** | W | 10.4 | 11.8 | 12.0 | **11.4** | 0.9 |
| **4** | W | 5.9 | 8.3 | 6.1 | **6.8** | 1.3 |
| **25** | W | 6.4 | 3.9 | 4.2 | **4.8** | 1.4 |

22

Table VI

| TPO-Rp (1-18, R9A, R11A) at 5 μM; "day 30" stability study | | | | | | |
|---|---|---|---|---|---|---|
| Condition | | STAT5 activation measured by protein phosphorylation (arbitrary units) | | | | |
| Temperature (°C) | Solvent | Exp #1 | Exp #2 | Exp #3 | Mean | SD |
| **-20** | W | 8.2 | 11.0 | 11.1 | **9.8** | 1.4 |
| **4** | W | 3.8 | 5.2 | 6.4 | **5.1** | 1.3 |
| **25** | W | 3.0 | 4.2 | 5.6 | **4.3** | 1.3 |
| **-20** | S | 6.1 | .8.2 | 7.4 | **7.2** | 1.1 |
| **4** | S | 4.6 | 5.8 | 6.2 | **5.5** | 0.8 |
| **25** | S | 1.8 | 4.2 | 3.2 | **3.1** | 1.2 |
| **-20** | H | 7.4 | 12.1 | 11.2 | **10.2** | 2.5 |
| **4** | H | 7.4 | 9.8 | 10.2 | **9.1** | 1.5 |
| **25** | H | 2.4 | 3.2 | 4.2 | **3.3** | 0.9 |

Table VII

| TPO-Rp (1-18, R9A, R11A) at 0.05 μM; "day 30" stability study | | | | | | |
|---|---|---|---|---|---|---|
| Condition | | STAT5 activation measured by protein phosphorylation (arbitrary units) | | | | |
| Temperature (°C) | Solvent | Exp #1 | Exp #2 | Exp #3 | Mean | SD |
| **-20** | W | 3.4 | 4.8 | 2.5 | **3.6** | 1.2 |
| **4** | W | 3.2 | 2.1 | 3.8 | **3.0** | 0.9 |
| **25** | W | 3.1 | 2.0 | 1.9 | **2.3** | 0.7 |
| **-20** | S | 2.2 | 1.9 | 2.5 | **2.2** | 0.3 |
| **4** | S | 3.2 | 2.1 | 1.8 | **2.4** | 0.7 |
| **25** | S | 2.2 | 1.8 | 1.9 | **2.0** | 0.2 |
| **-20** | H | 6.2 | 4.5 | 5.9 | **5.5** | 0.9 |
| **4** | H | 5.4 | 4.2 | 6.1 | **5.2** | 1.0 |
| **25** | H | 2.1 | 1.5 | 1.9 | **1.8** | 0.3 |

Table VIII

| TPO-Rp (wild type) at 5 μM; "day 30" stability study | | | | | | |
|---|---|---|---|---|---|---|
| Condition | | STAT5 activation measured by protein phosphorylation (arbitrary units) | | | | |
| Temperature (°C) | Solvent | Exp #1 | Exp #2 | Exp #3 | Mean | SD |
| **-20** | W | 4.9 | 6.5 | 3.2 | **4.9** | 1.7 |
| **-20** | S | 6.1 | 5.2 | 7.4 | **6.2** | 1.1 |
| **-20** | H | 6.4 | 5.2 | 8.5 | **6.7** | 1.7 |

Table IX

| TPO-Rp (wild type) at 0.05 μM; "day 30" stability study | | | | | | |
|---|---|---|---|---|---|---|
| Condition | | STAT5 activation measured by protein phosphorylation (arbitrary units) | | | | |
| Temperature (°C) | Solvent | Exp #1 | Exp #2 | Exp #3 | Mean | SD |
| **-20** | W | 3.2 | 2.1 | 1.5 | **2.3** | 0.9 |
| **-20** | S | 2.9 | 3.1 | 1.2 | **2.4** | 1.0 |
| **-20** | H | 4.2 | 2.4 | 2.9 | **3.2** | 0.9 |

Table X

| Approximate EC$_{50}$ values for shortened TPO-Rp peptides. | |
|---|---|
| **Peptide** | **Approximate EC$_{50}$ value*** |
| wild type | 80 nM |
| A1-L18 | 90 nM |
| **A1-L18 (R9A, R11A)** | **10 nM** |
| G4-L18 | 70 nM |
| G4-L18 (R9A, R11A) | 90 nM |
| G4-R21 | 80 nM |
| G4-R21 (R9A, R11A) | 80 nM |
| Y14F | 100 nM |
| *• Measuements of STAT5 phosphorylation* | |

## Claims

1. An oligopeptide with the biological activity of a TPO (thrombopoietin) receptor modulator comprising 15 to 18 amino acids having the general formula

$$X_1 \; G \; T \; L \; E \; L \; X_2 \; P \; X_3 \; S \; R \; Y \; R \; L \; Q \; L \; X_4,$$

wherein

X$_1$ is A R G or is missing,

X$_2$ is R or A,

X$_3$ is R or A and

X$_4$ is R A R or is missing

or a biological equivalent thereof.

2. The oligopeptide according to claim 1, which is selected from the group consisting of any one of the amino acid sequences defined in SEQ ID Nos. 1 to 6 or biological equivalents thereof.

3. An oligopeptide with the amino acid sequence defined in SEQ ID No. 7.

4. A nucleotide sequence encoding the oligopeptide according to claim 1, 2 or 3.

5. A vector comprising the nucleotide sequence of claim 4.

6. The vector according to claim 5, which is a bacterial, viral, mammalian or yeast vector.

7. The vector according to any one of claims 5 or 6 further comprising 5' and/or 3' regulatory elements capable of directing expression of the nucleotide sequence in a suitable host cell.

8. A host cell comprising the vector of any claims 5 to 7 being capable of expressing the bligopeptide of claim 1 or 2 under suitable conditions.

9. The host cell of claim 8, which is a mammalian, yeast or bacterial cell.

**10.** A method for genetically modifying a cell by transfecting the cell with a vector according to any one of claims 5 to 7.

**11.** The method of claim 10, whereby the cell is transfected by chemically or electrically induced transfection, in particular electroporation or cell fusion, retrovirus or virus mediated gene transfer, liposome mediated gene transfer or particle bombardment.

**12.** A method for producing a non-human mammalian animal capable of forming an oligopeptide according to claim 1, 2 or 3, wherein a nucleotide sequence according to claim 4 is introduced into a non-human mammalian animal cell.

**13.** A non-human mammalian animal comprising in its germ cells and/or somatic cells a nucleotide sequence of claim 4, comprising in particular a host cell according to claims 8 or 9.

**14.** The non-human mammalian animal of claim 13, which is a rodent or primate.

**15.** A method for the preparation of an oligopeptide according to claims 1, 2 or 3 comprising transfecting a cell with a vector according to any one of claims 5 to 7, culturing the cell in a culture medium under conditions allowing the expression of the oligopeptide and recovering - the oligopeptide from the cell or culture medium.

**16.** An antibody specifically binding to the oligopeptide of claim 1, 2 or 3.

**17.** The antibody of claim 16, which is a monoclonal or polyclonal antibody or a fragment thereof.

**18.** An antibody specifically binding to the antibody of claim 16 or 17.

**19.** A pharmaceutical composition for the treatment of hematological disorders, in particular thrombocytopenia comprising the oligopeptide of claim 1, 2 or 3, the nucleotide sequence of claim 4, the vector of any one of claims 5 to 7, the host cell of claim 8 or 9 or the antibody of claim 16, 17 or 18 optionally in conjunction with a pharmaceutically acceptable carrier.

**20.** A diagnostic composition for the diagnosis of hematological disorders, in particular thrombocytopenia comprising the oligopeptide of claims 1, 2 or 3, the nucleotide sequence of claim 4, the vector of any one of claims 5 to 7, the host cell of claims 8 or 9 or the antibody of claims 16, 17 or 18 optionally in conjunction with a pharmaceutically acceptable carrier.

**21.** The pharmaceutical or diagnostic composition of claim 19 or 20 further comprising thrombopoietin.

**22.** The pharmaceutical or diagnostic composition of any one of claims 19 to 21, which is in the form of a tablet, a pill, a capsule, granules, a suppository, a powder, a patch, a liposome, a coating, a solution for injection, infusion or oral administration, a syrup, a suspension, an emulsion, a spray, an inhalate, an aerosol, a paste, a salve or a lotion.

**23.** The use of the oligopeptide of claims 1, 2 or 3, the nucleotide sequence of claim 4, the vector of any one of claims 5 to 7, the host cell of claims 8 or 9 and/or the antibody or 16, 17 or 18 for the preparation of a medicament for diagnosing or treating hematological disorders, in particular thrombocytopenia.

**24.** The use of claim 23, wherein the medicament further comprises thrombopoietin.

**25.** A method for modulating the activity of a TPO-R (thrombopoietin-receptor), wherein the oligopeptide of claims 1, 2 or 3 or the antibody of claims 16, 17 or 18 is applied to TPO-R in the absence or presence of thrombopoietin.

**26.** The method of claim 25, wherein the modulation is an increase or decrease in activity.

**27.** A method for screening drugs effective in diagnosing or treating hematological disorders, in particular thrombocytopenia comprising screening potential drugs for their ability to compete with the oligopeptides of claim 1, 2 or 3 for binding to TPO-R or for activating intracellular signalling pathways.

**28.** A method for treating a patient suffering from a disorder that is susceptible to treatment with a thrombopoietin agonist, comprising administering to the patient a therapeutically effective dose or amount of an oligopeptide of claim 1, 2 or 3 and/or of the wild type TPO-Rp.

29. The method of claim 27, wherein the disorders are hematological disorders or thrombocytopenia resulting from bone marrow transfusions, radiation therapy, chemotherapy, allergic reactions or are idiopathic.

30. The method of claim 28 or 29, comprising further administering thrombopoietin.

31. Use of wild type TPO-Rp, the nucleotide sequence encoding wild type TPO-Rp, a vector comprising the nucleotide sequence, a host cell comprising the vector and/or an antibody specifically binding to wild type TPO-Rp for preparing a medicament for treating hematological disorders, in particular thrombocytopenia.

32. A method for detecting or isolating TPO-R from a TPO-R containing source comprising applying the oligopeptide of claim 1, 2 or 3 to the TPO-R containing source under conditions allowing binding of TPO-R to the oligopeptides and isolating TPO-R therefrom.

33. An immunoassay for detecting and/or isolating the oligopeptides of claims 1 to 3 from a mixture wherein the antibodies of claims 16 or 17 are applied to the mixture and the oligopeptides bound to the antibodies are detected and/or isolated.

EFFECT OF TPO/Rp (1-18;9,11-Ala) ON PLATELETS IN
CARBOPLATIN-TREATED 8 WEEK-OLD MALE C57BL/6J MICE

Fig. 1

EFFECT OF TPO/RCN01303 ON PLATELETS IN
CARBOPLATIN-TREATED 8 WEEK-OLD MALE C57BL/6J MICE

Fig. 2

## TPORp And Analog Stability Study -- Day Zero

*Thirty nmole Of A 1mM Solution*
*Reconstituted From A Dry Powder*

Fig. 3

## TPORp And Analog Stability Study -- Day Two
*Samples Stored As A 1mM Solution*

Fig. 4A

# TPORp And Analog Stability Study -- Day Two

*Samples Stored As A Dry Powder*

Fig. 4B

# TPORp And Analog Stability Study -- Day Nine

### Samples Stored As A 1mM Solution

Fig. 5A

TPORp And Analog Stability Study -- Day Nine

*Samples Stored As A Dry Powder*

Fig.5B

Fig. 6A

# TPORp And Analog Stability Study -- Day Fourteen

## *Samples Stored As A Dry Powder*

Fig. 6B

**European Patent Office**

**PARTIAL EUROPEAN SEARCH REPORT**

Application Number

which under Rule 45 of the European Patent Convention shall be considered, for the purposes of subsequent proceedings, as the European search report

EP 00 10 8075

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WO 99 42127 A (RECEPTRON INC) 26 August 1999 (1999-08-26) * sequence ID no.10 * * page 17, line 18 - line 19; claims * | 1,3,4, 19-33 | C12N15/12 C07K14/715 C07K16/28 A01K67/027 |
| A | * page 21 - page 25 * * page 4, line 6 - page 5, line 12 * * page 7, line 6 - line 28; figures 7-9 * * page 43, line 1 * ——— | 5-18 | A61K48/00 A61K38/17 A61K38/10 A61K31/70 A61K39/395 |
| A | WO 99 10494 A (GENENTECH INC) 4 March 1999 (1999-03-04) * claims * ——— | 1,16-18 | G01N33/68 C12Q1/68 |
| A | WO 95 19992 A (AMGEN INC) 27 July 1995 (1995-07-27) * claims; examples * ——— -/-- | 1-33 | |

**TECHNICAL FIELDS SEARCHED (Int.Cl.7)**

C07K
C12N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 3 October 2000 | Le Cornec, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**INCOMPLETE SEARCH
SHEET C**

Application Number

EP 00 10 8075

Although claims 10-11, 25-26 (as far as they concern an in vivo method) and claims 28-30 are directed to a method of treatment of the human/animal body (Article 52(4) EPC), the search has been carried out and based on the alleged effects of the compound/composition.

**European Patent Office**    **PARTIAL EUROPEAN SEARCH REPORT**    **Application Number**

EP 00 10 8075

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| D,A | VIGON I ET AL: "MOLECULAR CLONING AND CHARACTERIZATION OF MPL, THE HUMAN HOMOLOG OF THE V-MPL ONCOGENE: IDENTIFICATION OF A MEMBER OF THE HEMATOPOIETIC GROWTH FACTOR RECEPTOR SUPERFAMILY" PROCEEDINGS OF THE NATIONAL ACADEMY OF SCIENCES OF USA,US,NATIONAL ACADEMY OF SCIENCE. WASHINGTON, vol. 89, June 1992 (1992-06), pages 5640-5644, XP002915951 ISSN: 0027-8424 | | |
| A | WO 96 40189 A (GLAXO GROUP LTD ;DOWER WILLIAM J (US); BARRETT RONALD W (US); CWIR) 19 December 1996 (1996-12-19) * abstract; claims * | | TECHNICAL FIELDS SEARCHED (Int.Cl.7) |
| D,A | CWIRLA S E ET AL: "PEPTIDE AGONIST OF THE THROMBOPOIETIN RECEPTOR AS POTENT AS THE NATURAL CYTOKINE" SCIENCE,AMERICAN ASSOCIATION FOR THE ADVANCEMENT OF SCIENCE,,US, vol. 276, 13 June 1997 (1997-06-13), pages 1696-1699, XP002067303 ISSN: 0036-8075 * the whole document * | 1-33 | |

## ANNEX TO THE EUROPEAN SEARCH REPORT
## ON EUROPEAN PATENT APPLICATION NO.

EP 00 10 8075

This annex lists the patent family members relating to the patent documents cited in the above-mentioned European search report.
The members are as contained in the European Patent Office EDP file on
The European Patent Office is in no way liable for these particulars which are merely given for the purpose of information.

03-10-2000

| Patent document cited in search report | | Publication date | Patent family member(s) | | Publication date |
|---|---|---|---|---|---|
| WO 9942127 | A | 26-08-1999 | AU | 2783099 A | 06-09-1999 |
| WO 9910494 | A | 04-03-1999 | AU | 8831298 A | 16-03-1999 |
| | | | EP | 1009831 A | 21-06-2000 |
| WO 9519992 | A | 27-07-1995 | US | 5498599 A | 12-03-1996 |
| | | | AT | 179992 T | 15-05-1999 |
| | | | AU | 682581 B | 09-10-1997 |
| | | | AU | 1832495 A | 08-08-1995 |
| | | | CA | 2181566 A | 27-07-1995 |
| | | | CN | 1143969 A | 26-02-1997 |
| | | | DE | 69509631 D | 17-06-1999 |
| | | | DE | 69509631 T | 30-12-1999 |
| | | | EP | 0739354 A | 30-10-1996 |
| | | | ES | 2131308 T | 16-07-1999 |
| | | | GR | 3030262 T | 31-08-1999 |
| | | | JP | 2853905 B | 03-02-1999 |
| | | | JP | 9502451 T | 11-03-1997 |
| | | | KR | 222274 B | 01-10-1999 |
| | | | NZ | 281395 A | 19-12-1997 |
| | | | ZA | 9500526 A | 12-12-1995 |
| WO 9640189 | A | 19-12-1996 | AU | 6046696 A | 30-12-1996 |
| | | | HR | 960256 A | 28-02-1998 |
| | | | AU | 6381896 A | 30-12-1996 |
| | | | EP | 0842293 A | 20-05-1998 |
| | | | WO | 9640987 A | 19-12-1996 |
| | | | AU | 704215 B | 15-04-1999 |
| | | | AU | 6163496 A | 30-12-1996 |
| | | | BR | 9608587 A | 05-01-1999 |
| | | | CA | 2223449 A | 19-12-1996 |
| | | | CN | 1192749 A | 09-09-1998 |
| | | | CZ | 9703897 A | 17-06-1998 |
| | | | EP | 0885242 A | 23-12-1998 |
| | | | HU | 9900921 A | 28-07-1999 |
| | | | JP | 3059218 B | 04-07-2000 |
| | | | JP | 10507776 T | 28-07-1998 |
| | | | NO | 975705 A | 05-02-1998 |
| | | | NZ | 310778 A | 28-10-1999 |
| | | | PL | 323917 A | 27-04-1998 |
| | | | WO | 9640750 A | 19-12-1996 |
| | | | US | 6121238 A | 19-09-2000 |
| | | | US | 5869451 A | 09-02-1999 |
| | | | US | 6083913 A | 04-07-2000 |

EPO FORM P0459

For more details about this annex : see Official Journal of the European Patent Office, No. 12/82